# EUROPEAN PATENT APPLICATION

(11) **EP 3 798 250 A1**
(43) Date of publication of application: **31.03.2021**
(21) Application number: 19199699.0
(22) Date of filing: 25.09.2019
(51) Int. Cl.: C08G 73/02, A61K 48/00, C08G 63/685, C12N 15/87, C08G 83/00

(54) **HYPERBRANCHED CATIONIC POLYMERS USEFUL AS NUCLEIC ACID DELIVERY VECTORS FOR TRANSFECTING**

(71) Applicant: University College Dublin, Belfield, Dublin 4 (IE)
(72) Inventor: The designation of the inventor has not yet been filed
(74) Representative: Purdylucey Intellectual Property

(57) **Abstract**

Hyperbranched cationic polymers are described. The polymers employ a 4-branching monomer resulting in an increase in the number of functional terminal groups due to the extra branching units, providing excellent transfection efficiency and cytocompatibility in different cell types, including aADSC, HeLa, Neu7 and RDEB keratinocytes, and delivering different genetic therapy approaches such GFP plasmid DNA and a ribonucleoprotein CRISP-Cas 9 complex for COL7A1 exon 80 skipping. In addition, the extra branching units of the polymer of the invention increases the positive charge on the polymer, which provides for improved endosomal escape within the cell. The 4-branching unit can be a diamine component, or a tetraacrylate component, although other 4-branching monomers may be employed such as for example any component with tetra acrylamide groups (i.e. 4-arm PEG acrylamide, 4-arm PEG maleimide), any component with tetra N-hydroxysuccinimide (NHS) groups (i.e. 4-arm PEG-succinimidyl carbonate NHS ester), any type of tetrathiol component (i.e. Pentaerythritol tetrakis(3-mercaptopropionate), 4-arm PEG-thiol, Tetra(2- mercaptoethyl)silane), and any tetraepoxy component (i.e. TetraGlycidyl methylenedianiline, Tetraglycidyl 1,1'-methylenebis(naphthalene-2,7-diol), Pentaerythritol tetraglycidyl ether, 4-arm peg epoxide).

## Description

### Field of the Invention

The present invention relates to hyperbranched polymers. Also contemplated are compositions comprising hyperbranched polymers complexed with a nucleic acid, and uses of the compositions to transfect cells.

### Background to the Invention

Following almost three decades of development, gene therapy has become a predominant part of the rapidly increasing armamentarium of nanomedicine for improving health conditions and correcting genetic disorders. Although multiple clinical trials using viral gene delivery vectors have been carried out, the risks of triggering immunogenic responses and transgene insertional mutagenesis, limitations associated with large-scale production and low "cargo capacity" for genetic materials, along with the unpredictability of vector mobility remain unaddressed. The only gene therapy approved in the history has been Glybera, a viral gene therapy to reverse lipoprotein lipase deficiency (LPDL). It was approved by EMA but the FDA required more long term studies not able to be completed by uniQure. The product was finally removed to the market due to the 1M cost per treatment.

From this perspective, non-viral gene delivery vectors would be more promising because of their potential for minimal immunogenicity, non-tumorigenicity, cost-effective manufacturing, high payload of nucleic acids and localized gene expression. The most common non-viral methods include expensive physical ones like the gene gun, electroporation and injection and relatively inefficient and toxic chemical formulations. The transfection efficiency for instance, only 27% and 44% of enhanced green fluorescence protein (EGFP) delivery efficiencies in human dermal fibroblast and human primary fibroblasts were detected by different electroporation systems. The maximum transfection efficiency of the leading cationic lipid reagents TransFectin, Lipofectanmmine LTX and electroporation in the mouse embryonic fibroblast was 15.7%, 11.8% and 48.1%, respectively. Also, those physical methods, besides expensive, are limited to and ex vivo use due to the impossibility of carrying on those techniques in vivo, so their use is really limited then.

Cationic liposome DNA complexes (lipoplexes) are the most commonly studied non-viral system, however, cationic polymeric DNA complexes (polyplexes) were found to be more stable. Cationic polymers are an attractive alternative given their facile synthesis, versatility and improved safety profile compared to viruses. Non-viral vectors can condense nucleic acids into nanoparticles via electrostatic interactions to protect them from enzymatic degradation and facilitate transport across the cell membrane in an efficient and cytocompatible manner. Clinical translation of polymer based non-viral gene delivery systems have been limited to date owing to both poor transfection efficiency and transgene expression. The first clinical trial using polymers for gene therapy dates back to 2010, with cyclodextrin being used to deliver siRNA for cancer treatment. Among the polymer-based gene therapy options, some clinical trials such the off-the-shelf cationic polymer polyethylenimine (PEI) has showed some promises. However, PEI is nondegradable and severely hampered by its safety concerns. Since then, a number of polymer-based clinical trials have been untaken, predominantly using PEI derivatives, yet none have obtained FDA approval on account of their failure to meet their primary endpoints (Davis, 2009, Chen et al., 2016). Despite this apparent failure, it demonstrates that clinical translation of polymer-based gene therapy is merely awaiting the development of an efficient delivery vector.

Linear poly(β-amino ester)s (LPAEs) have emerged as one type of the most versatile gene delivery vectors. Since they were first developed in 2000 by Lynn and Langer, more than 2500 LPAEs have been designed, synthesized, and screened by Green et al., and Anderson et al. The best-performing C32 series have been identified for both in vitro and in vivo gene delivery, which can even rival adenovirus on human umbilical vein endothelia cells. To date, most design, synthesis, and transfection studies with poly(β-amino ester)s have been focused on their linear structure. Although the results from LPAEs are very encouraging, the potential for synthesizing and optimizing structures with multiple functional groups is limited by the linear nature of these polymers.

WO2016/020474 describes highly branched poly(b-amino ester)s formed by reacting together a 3-branching triacrylate component, a diacrylate component, and a primary amine component to form a polymer, and then reacting the polymer with primary or secondary amine endcapping monomers to provide a highly branched cationic polymer. This polymer was found to provide for enhanced gene transfection efficiency compared with linear poly(b-amino ester)s.

It is an object of the invention to overcome at least one of the above-referenced problems.

### Summary of the Invention

The Applicant provides a highly branched cationic polymer that employs a 4-branching monomer resulting in an increase in the number of functional terminal groups due to the extra branching units, providing excellent transfection efficiency and cytocompatibility in different cell types, including aADSC, HeLa, Neu7 and RDEB keratinocytes, and delivering different genetic therapy approaches such GFP plasmid DNA and a ribonucleoprotein CRISP-Cas 9 complex for COL7A1 exon 80 skipping. In addition, the extra branching units of the polymer of the invention increases the positive charge on the polymer, which provides for improved endosomal escape within the cell. As described herein, the 4-branching unit can be a diamine component, or a tetraacrylate component, although other 4-branching monomers may be employed such as for example any component with tetra acrylamide groups (i.e. 4-arm PEG acrylamide, 4-arm PEG maleimide), any component with tetra N-hydroxysuccinimide (NHS) groups (i.e. 4-arm PEG-succinimidyl carbonate NHS ester), any type of tetrathiol component (i.e. Pentaerythritol tetrakis(3-mercaptopropionate), 4-arm PEG-thiol, Tetra(2- mercaptoethyl)silane), and any tetraepoxy component (i.e. TetraGlycidyl methylenedianiline, Tetraglycidyl 1,1'-methylenebis(naphthalene-2,7-diol), Pentaerythritol tetraglycidyl ether, 4-arm peg epoxide).

In a first aspect, the invention provides a polymer made by reacting together:
(i) a four-branching monomer having four reaction sites that can react with acrylate or amine groups;
(ii) a diacrylate component, typically of formula (I) 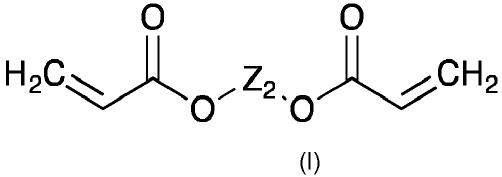
   wherein Z2 is a linear or branched carbon chain of 1 to 30 carbon atoms, a linear or branched heteroatom-containing carbon chains of 1 to 30 atoms, a carbocycle containing 3 to 30 carbon atoms, or a heterocycle containing 3 to 30 atoms;
   wherein Z2 is unsubstituted or substituted with at least one of a halogen, a hydroxyl, an amino group, a suifonyl group, a sulphonamide group, a thiol, a C1-C6 alkyl, a C1-C0 alkoxy, a C1-C6 ether, a C1-C6 thioether, a C1-C6 sulfone, a C1-C6 sulfoxide, a C1-C6 primary amide, a C1-C6 secondary amide, a halo C1-C5 alkyl, a carboxyl group, a cyano group, a nitro group, a nitroso group, -OC(0)NR'R', -N(R')C(0)NR'R, -N(R')C(0)0-C1-C6 alkyl, C3-C6 cycloalkyl, C3-C6 heterocyclyl, C2-C5 heteroaryl and C6-C10 aryl; wherein each R' is independently selected, from the group consisting of hydrogen and Ci-C6 alkyl;
(iii) a first amine component typically comprising 3 to 20 atoms,
   wherein said amine component typically is unsubstituted or substituted with at least one of a halogen, a hydroxyl, an amino group, a sulfonyl group, a sulphonamide group, a thiol, a C1-C6 alkyl, a C1-C6 alkoxy, a C1-C6 ether, a C1-C6 thioether, a C1-C6 sulfone, a C1-C6 sulfoxide, a C1-C6 primary amide, a C1-C6 secondary amide, a halo C1-C6 alkyl, a carboxyl group, a cyano group, a nitro group, a nitroso group, -OC(0)NR'R', - N(R')C(0)NR'R, -N(R')C(0)0-C1-C6 alkyl, C3-C6 cycloalkyl, C3-C6 heterocyclyl, C2-C5 heteroaryl and C6-C10 aryl; wherein each R' is independently selected, from the group consisting of hydrogen and C1-C6 alkyl; and
(iv) a second amine component typically comprising 3 to 20 atoms,
   wherein said amine component typically is unsubstituted or substituted with at least one of a halogen, a hydroxyl, an amino group, a sulfonyl group, a sulphonamide group, a thiol, a C1-C6 alkyl, a C1-C6 alkoxy, a C1-C6 ether, a C1-C6 thioether, a C1-C6 sulfone, a C1-C6 sulfoxide, a C1-C6 primary amide, a C1-C6 secondary amide, a halo C1-C6 alkyl, a carboxyl group, a cyano group, a nitro group, a nitroso group, -OC(0)NR'R', - N(R')C(0)NR'R, -N(R')C(0)0-C1-C6 alkyl, C3-C6 cycloalkyl, C3-C6 heterocyclyl, C2-C5 heteroaryl and C6-C10 aryl; wherein each R' is independently selected, from the group consisting of hydrogen and C1-C6 alkyl.

In another aspect, there is provided a method of forming a polymer comprising a step of reacting together:
(i) a four-branching monomer;
(ii) a diacrylate component, typically of formula (I) 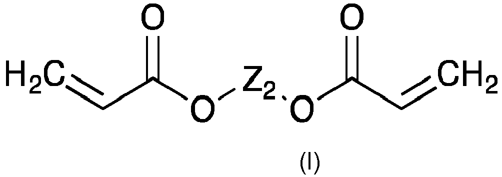
   wherein Z2 is a linear or branched carbon chain of 1 to 30 carbon atoms, a linear or branched heteroatom-containing carbon chains of 1 to 30 atoms, a carbocycle containing 3 to 30 carbon atoms, or a heterocycle containing 3 to 30 atoms;
   wherein Z2 is unsubstituted or substituted with at least one of a halogen, a hydroxyl, an amino group, a suifonyl group, a sulphonamide group, a thiol, a C1-C6 alkyl, a C1-C0 alkoxy, a C1-C6 ether, a C1-C6 thioether, a C1-C6 sulfone, a C1-C6 sulfoxide, a C1-C6 primary amide, a C1-C6 secondary amide, a halo C1-C5 alkyl, a carboxyl group, a cyano group, a nitro group, a nitroso group, -OC(0)NR'R', -N(R')C(0)NR'R, -N(R')C(0)0-C1-C6 alkyl, C3-C6 cycloalkyl, C3-C6 heterocyclyl, C2-C5 heteroaryl and C6-C10 aryl; wherein each R' is independently selected, from the group consisting of hydrogen and Ci-C6 alkyl
(iii) a first amine component typically comprising 3 to 20 atoms,
   wherein said amine component typically is unsubstituted or substituted with at least one of a halogen, a hydroxyl, an amino group, a sulfonyl group, a sulphonamide group, a thiol, a C1-C6 alkyl, a C1-C6 alkoxy, a C1-C6 ether, a C1-C6 thioether, a C1-C6 sulfone, a C1-C6 sulfoxide, a C1-C6 primary amide, a C1-C6 secondary amide, a halo C1-C6 alkyl, a carboxyl group, a cyano group, a nitro group, a nitroso group, -OC(0)NR'R', - N(R')C(0)NR'R, -N(R')C(0)0-C1-C6 alkyl, C3-C6 cycloalkyl, C3-C6 heterocyclyl, C2-C5 heteroaryl and C6-C10 aryl; wherein each R' is independently selected, from the group consisting of hydrogen and C1-C6 alkyl; and
(iv) a second amine component typically comprising 3 to 20 atoms,
   wherein said amine component typically is unsubstituted or substituted with at least one of a halogen, a hydroxyl, an amino group, a sulfonyl group, a sulphonamide group, a thiol, a C1-C6 alkyl, a C1-C6 alkoxy, a C1-C6 ether, a C1-C6 thioether, a C1-C6 sulfone, a C1-C6 sulfoxide, a C1-C6 primary amide, a C1-C6 secondary amide, a halo C1-C6 alkyl, a carboxyl group, a cyano group, a nitro group, a nitroso group, -OC(0)NR'R', - N(R')C(0)NR'R, -N(R')C(0)0-C1-C6 alkyl, C3-C6 cycloalkyl, C3-C6 heterocyclyl, C2-C5 heteroaryl and C6-C10 aryl; wherein each R' is independently selected, from the group consisting of hydrogen and C1-C6 alkyl.

In one embodiment, the polymer is formed using a oligomer combination approach, in which the diacrylate and first amine components are reacted together to form a first oligomer, the first oligomer and second amine component are reacted together to form a second oligomer, and the second oligomer and four branching monomer are reacted together to form the hyperbranched polymer of the invention. This oligomer combination approach is described in detail in Zeng et al (Nano. Lett. 2019 19, 381-391).

In another embodiment, the four-branching monomer, diacrylate component, and first amine are reacted together in a Michael Addition reaction to form a first polymer, and the frist polymer and second amine component (endcapping amine) are reacted together in a Michael Addition reaction to form the hyperbranched polymer of the invention.

In one embodiment, the four-branching monomer is selected from a diamine or a tetraacrylate component.

In one embodiment, the diamine component has a structure NH2-L4-NH2, in which L4 is a linker. The linker may be an aryl or heteroaryl group, for example C1-C10 aryl or heteroaryl group, including 0-5 heteroatoms selected from O, N and S.

In one embodiment, the diamine component is selected from:

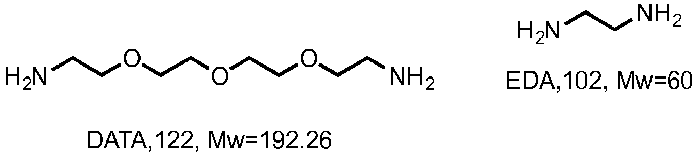

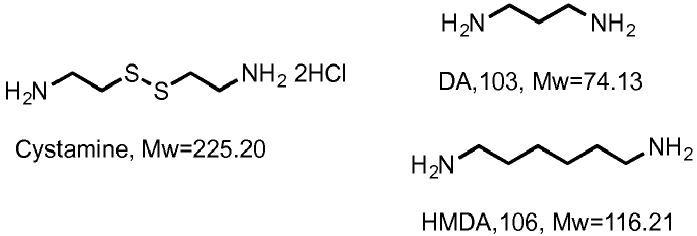

In one embodiment, the tetraacrylate component is a 4-arm PEG acrylate or has a formula (II), 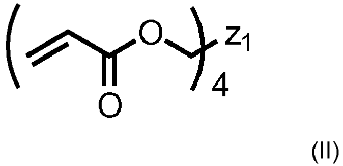 in which Z1 is a scaffold consisting of:
a linear or branched carbon chain of 1 to 30 carbon atoms, a linear or branched heteroatom-containing carbon chains of 1 to 30 atoms, a carbocycle containing 3 to 30 carbon atoms, or a heterocycle containing 3 to 30 atoms;
wherein Z1 is unsubstituted or substituted with at least one of a halogen, a hydroxyl, an amino group, a sulfonyl group, a sulphonamide group, a thiol, a C1-C6 alkyl, a C1-C6 alkoxy, a C1-C6 ether, a C1-C6 thioether, a C1-C6 sulfone, a Ci-C6 sulfoxide, a C1-C6 primary amide, a C1-C6 secondary amide, a halo C1-C6 alkyl, a carboxyl group, a cyano group, a nitro group, a nitroso group, -OC(0)NR'R', -N(R')C(0)NR'R, -N(R')C(0)0-C1-C6 alkyl, C3-C6 cycloalkyl, C3-C6 heterocyclyl, C2-C5 heteroaryl and C6-C10 aryl; wherein each R' is independently selected, from the group consisting of hydrogen and C1-C6 alkyl;

In one embodiment, the tetraacrylate component is selected from:

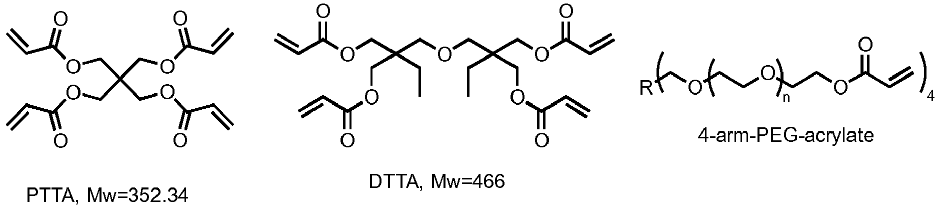

In one embodiment, the diacrylate component of formula (I) is selected from

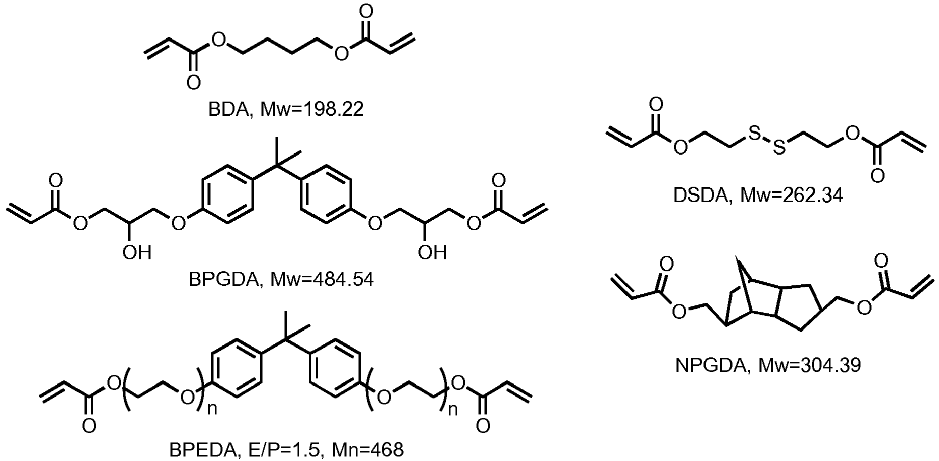

In one embodiment, the first amine component is selected from:

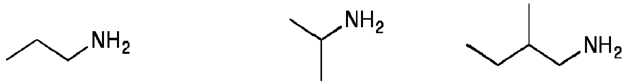

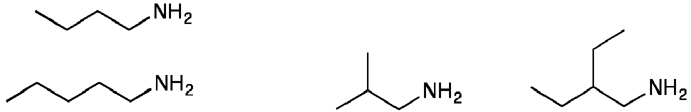

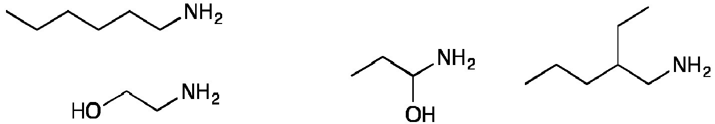

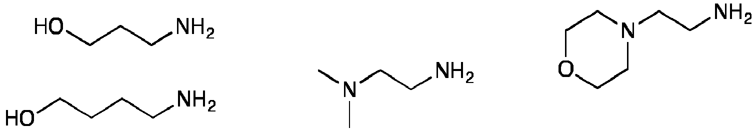

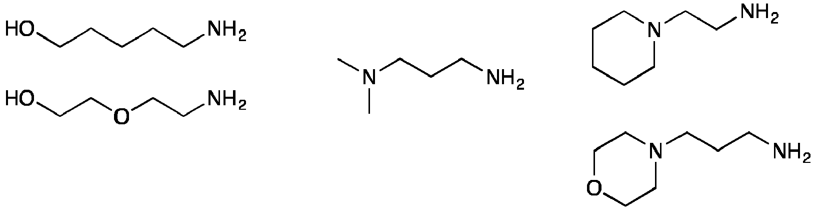

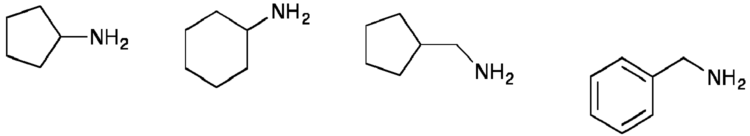

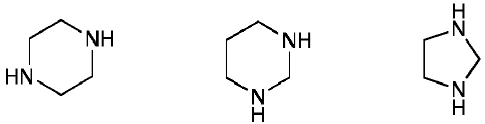

In one embodiment, the second amine component is a diamine component having a structure NH₂-L₄-NH₂ or is selected from:

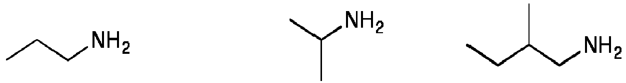

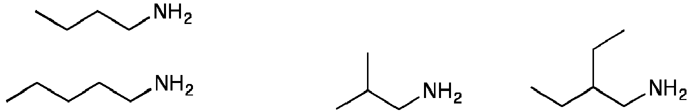

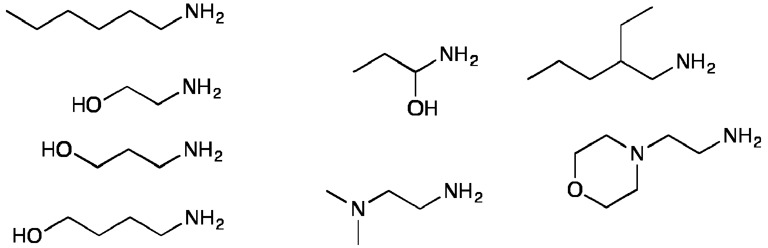

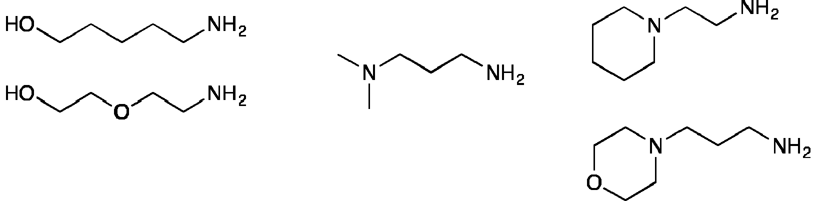

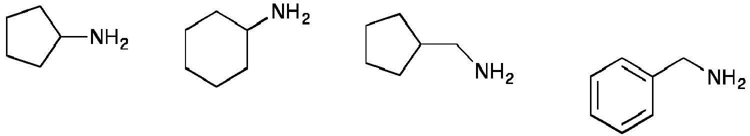

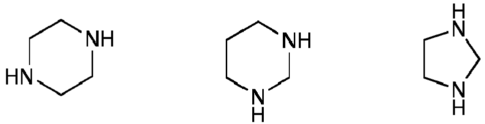

In a preferred embodiment, the second amine component is selected from:

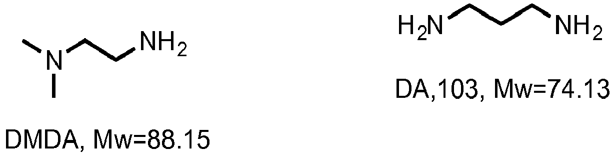

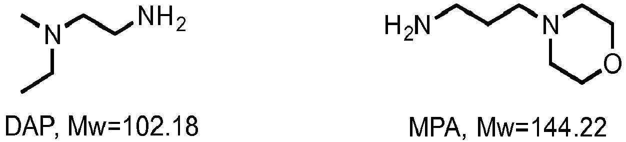

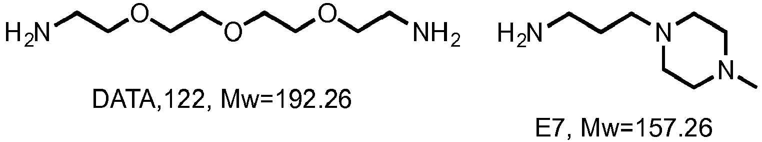

In another aspect, there is provided a composition comprising a hyperbranched polymer of the invention.

In one embodiment, the composition is a pharmaceutical composition and comprises a pharmaceutically acceptable excipient.

In one embodiment, the composition comprises a nucleic acid component complexed together with the polymer of the invention. The composition is referred to herein as a "polyplex". The nucleic acid component may be a nucleic acid, a complex of a nucleic acid and a protein/peptide or combination of proteins or peptides (ribonucleoprotein). In one embodiment, the nucleic acid component is a nucleic acid, a gene editing system, a plasmid, a minicircle, a nanoplasmid, or an expression vector. The nucleic acid may be DNA, RNA or a combination of both. RNA may be employed in a format consisting of but not limited to, siRNA, dsRNA, shRNA, microRNA, crRNA, tracRNA, sgRNA, mRNA, RNA oligonucleotides, antisense oligonucleotides. The gene editing system may be a CRISPR-associated Cas system (Sander and Joung (2014) CRISPR-Cas systems for editing, regulating and targeting genomes Nature Biotechnology 32(4): 347-355)), a TALEN system (Boch J (February 2011); "TALEs of genome targeting". Nature Biotechnology. 29 (2): 135-6. doi:10.1038/nbt.1767. PMID 21301438), a meganuclease system, or a zinc finger nuclease (ZFN) system (Carroll, D (2011) "Genome engineering with zinc-finger nucleases". Genetics Society of America. 188 (4): 773 78doi:10.1534/genetics.111.131433. PMC 3176093. PMID 21828278). In one embodiment, the gene editing system is configured to perform insertational mutagenesis on a cell, for example OBLIGARE systems, and CRISPR-Cpf1 systems (Maresca et al. (2013) Obligate Ligation-Gated Recombination (ObLiGaRe): Custom-designed nuclease-mediated targeted integration through nonhomologous end joining Genome Res. 23: 539-546; see also WO2014/033644), Fagerlund et al. (2015) The Cpf1 CRISPR-Cas protein expands genome-editing tools Genome Biology 16: 251-253; Ledford (2015) Bacteria yield new gene cutter Smaller CRISPR enzyme should simplify genome editing Nature 526: 17).

In one embodiment, the composition has a particulate form. In one embodiment, the particulate composition has a particle size of less than 2 µm, 1.5 µm, 1000 nm, for example 20-900 nm, 50-800 nm, 50-700 nm, 50-600 nm, 50-500 nm, 50-400 nm, 50-300 nm, 100-300 nm.

The compositions of the invention may be employed in gene addition, gene replacement, gene knockdown and gene editing. Gene replacement is defined as the provision of a functional healthy copy of a gene to replace a dysfunctional mutant containing gene which has given rise to a disease.Gene addition is defined as the supplementation of therapeutic genes that target a specific aspect of a disease mechanism.Gene knockdown is defined as the process of inhibiting a target genes capability to synthesize a toxic/dysfunctional protein which gives rise to a disease. Gene editing is defined as the process whereby a target genes nucleotide sequence is altered resulting in either a loss of function/correction/manipulation of gene expression. Such gene editing systems consists of but are not limited to i) clustered, regularly interspaced, palindromic repeats (CRISPR)-associated (Cas) system; (ii) a transcription activator-like effector nuclease (TALEN) system; or (iii) a zinc finger nuclease (ZFN) system.

Other applications include
1). Vector for research use
2.) If combined with a probe the invention can be used as a cell/tissue marker for research or diagnostic
3.) If combined with a pharmaceutical agent can be used as drug delivery system
4.) If combined with a targeting moeity can be used to target specific cell types
5.) If combined with a pharmaceutical agent and a targeting moiety can be used to deliver a drug in a specific organ, tissue or cell type.

In another aspect, there is provided a method of transfecting a cell comprising a step of contacting one or more target cells with a composition of the invention under conditions suitable for transfecting the cell with the composition. Transfection may be in-vivo, ex-vivo, or in-vitro. Transfection may involve modification of the genome of the cell (for example by deleting all or part of the genome), inserting a sequence into the genome (insertational mutagenesis), silencing a gene, replacing a gene, upregulating expression of a gene, editing a genome (for example deleting disease causing mutations), and adding residues required for proper functioning of the gene.

In another aspect, the invention relates to a cell transformed by a method of the invention.

Other aspects and preferred embodiments of the invention are defined and described in the other claims set out below.

### Brief Description of the Figures

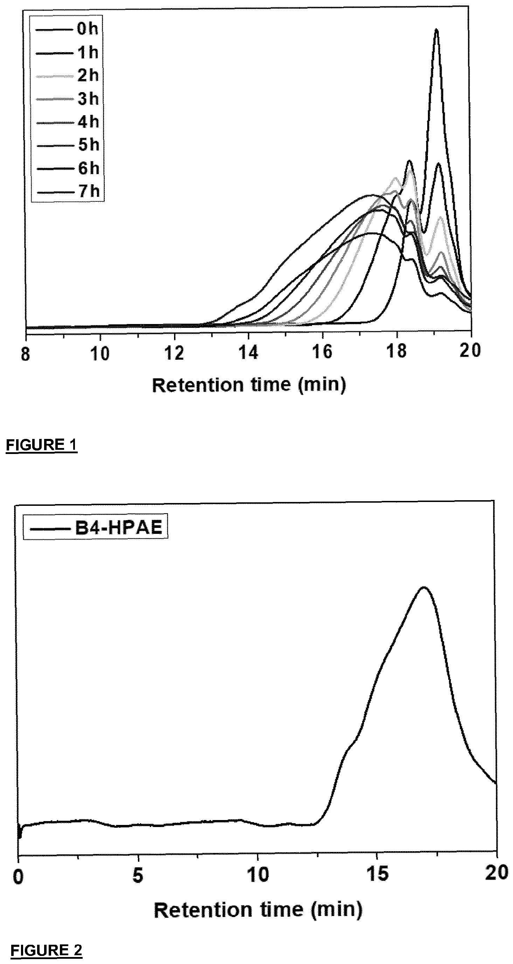

**FIGURE 1** shows the polymer chain propagation during the Michael addition polymerization process for the Y4 hyperbranched polymer. The monomer content is decreased as the polymer chain is growing.
**FIGURE 2** shows the purified GPC result of the Y4 hyperbranched polymer after 48 h of endcapping reaction.

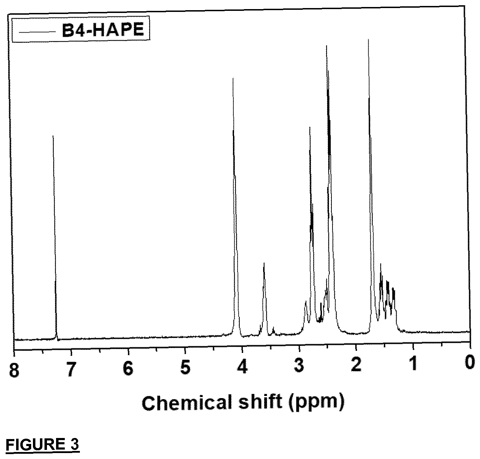

**FIGURE 3** shows the NMR spectrum of purified hyperbranched Y4 polymer. There is no acrylate peaks left which means all the terminal acrylate groups were converted into amine endcapped group.

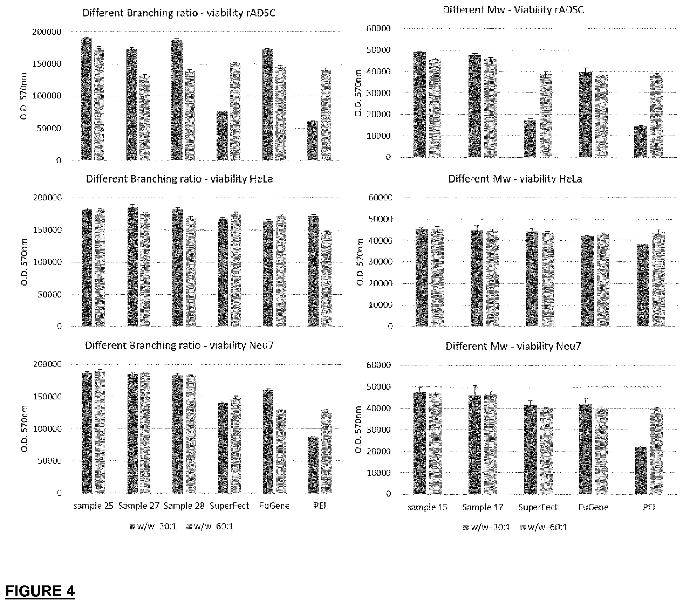

**FIGURE 4** shows that different branching ratios and different Mw of Y4 polymer induce higher cell viability than the commercial transfection vectors (SuperFect, FuGene and PEI) after transfection of different cell lines (rADSC, HeLa, and Neu7) with gWIZ plasmid at polymer/DNA ratios of 30:1 and 60:1 using AlamarBlue assay.

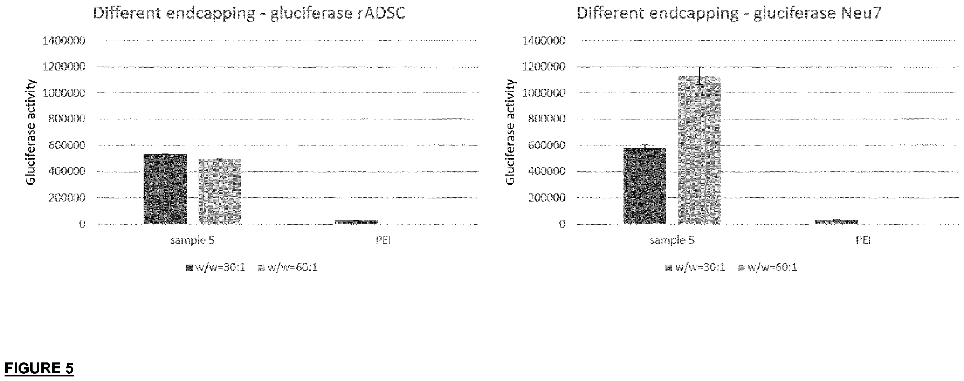

**FIGURE 5** shows the higher transfection efficiency induced by the polyplexes formed by Y4 polymers(with different endcapping monomers) and gWIZ plasmid at 30:1 and 60:1 ratio in comparison with the commercial PEI transfection vector, measured as gluciferase activity after transfection of rADSC and Neu7 cell lines.

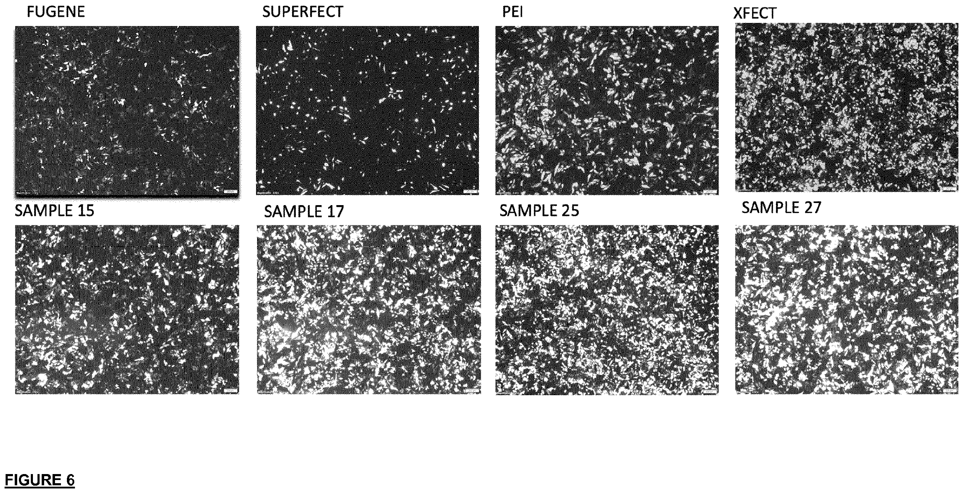

**FIGURE 6** shows the higher transfection efficiency for the Y4 polymers (samples 15, 17, 25, 27) with different branching ratios and Mw in comparison with the commercial transfection vectors FuGene, SuperFect, PEI and XFect by higher fluoresce emission of the GFP marker after transfection with a gWIZ plasmid.

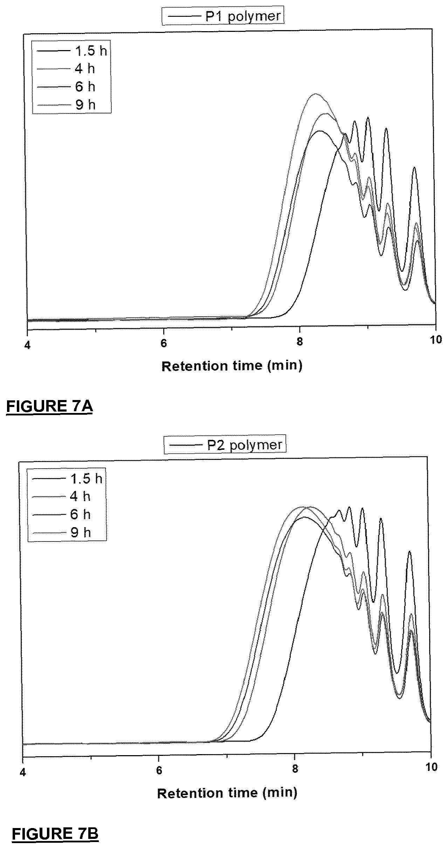

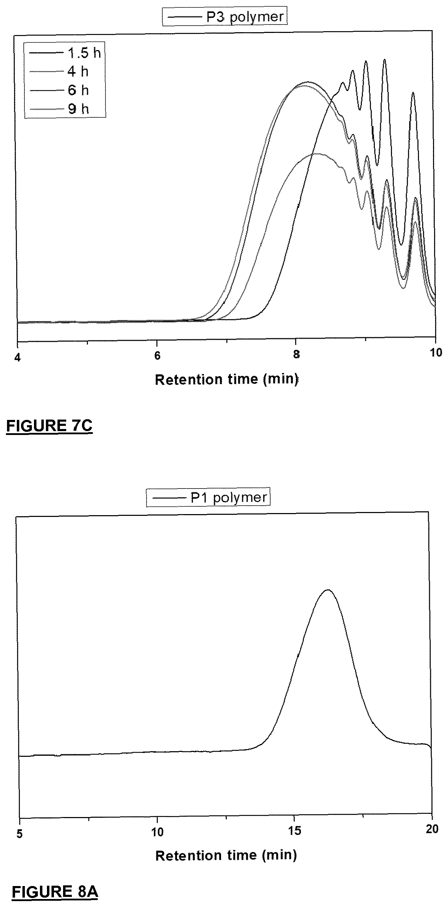

**FIGURE 7** shows the P1, P2, and P3 polymer chain propagation during the Michael addition polymerization process.

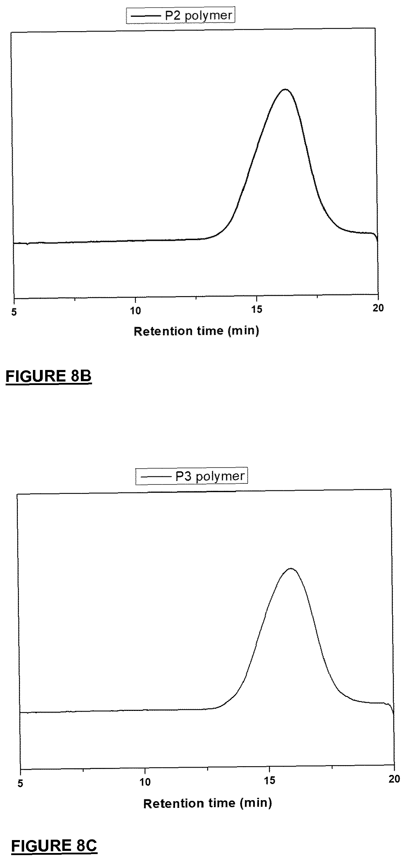

**FIGURE 8** shows the purified GPC result of P1, P2, and P3 polymers after 48 h of endcapping reaction.

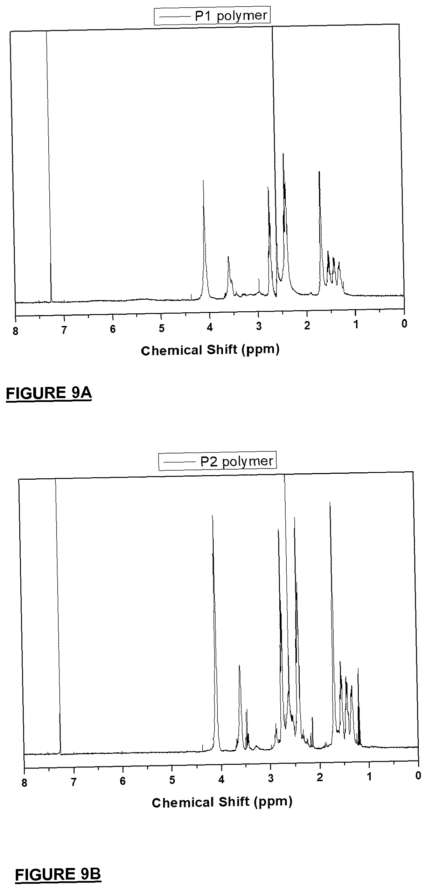

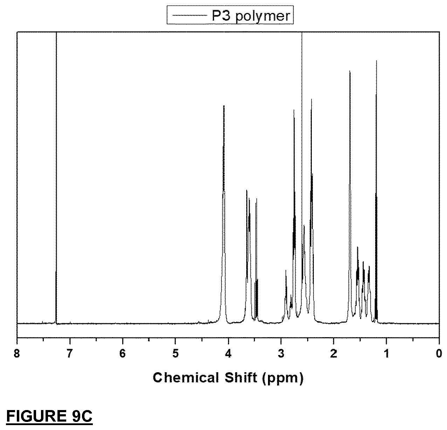

**FIGURE 9** shows the NMR spectra of purified P1, P2, and P3 polymers. There are no acrylate peaks left which means all the terminal acrylate groups were converted into amine endcapped group.

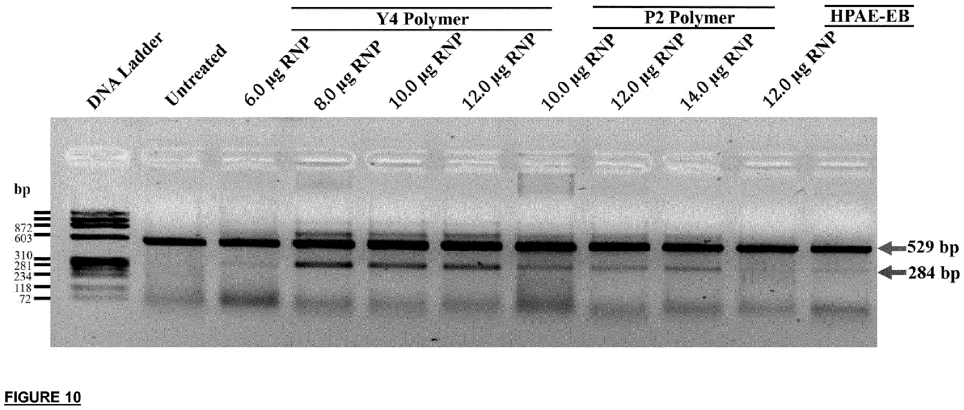

**FIGURE 10** Comparison of correction efficiency using the RNP system shows higher correction efficiency of polyplexes employing a hyperbranched polymer of the invention (Y4 and P2) compared with HPAE polymer.
**FIGURE 11** Proton Nuclear Magnetic Resonance (1H-NMR) Measurement of four-branching polymers of the invention:
   (A) Y polymer-BDA-S5-EDA-103 ; (B) Y polymer-BDA-S5-EDA-122; (C) Y polymer-BDA-S5-HMDA-103; (D) Y polymer-BDA-S5-HMDA-122; (E) P polymer-BDA-S5-PTTA-103 (F) P polymer-BDA-S5-PTTA-122; (G) P polymer-BDA-S5-DTTA-103; (H) P polymer-BDA-S5-DTTA-122
**FIGURE 12** Molecular Weight Determination of four-branching polymers of the invention by Gel Permeation Chromatography (GPC):
   (A) Y polymer-BDA-S5-EDA-103 ; (B) Y polymer-BDA-S5-EDA-122; (C) Y polymer-BDA-S5-HMDA-103; (D) Y polymer-BDA-S5-HMDA-122; (E) P polymer-BDA-S5-PTTA-103 (F) P polymer-BDA-S5-PTTA-122; (G) P polymer-BDA-S5-DTTA-103; (H) P polymer-BDA-S5-DTTA-122

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, age, poisoning or nutritional deficiencies.

As used herein, the term "treatment" or "treating" refers to an intervention (e.g. the administration of an agent to a subject) which cures, ameliorates or lessens the symptoms of a disease or removes (or lessens the impact of) its cause(s) (for example, the reduction in accumulation of pathological levels of lysosomal enzymes). In this case, the term is used synonymously with the term "therapy".

Additionally, the terms "treatment" or "treating" refers to an intervention (e.g. the administration of an agent to a subject) which prevents or delays the onset or progression of a disease or reduces (or eradicates) its incidence within a treated population. In this case, the term treatment is used synonymously with the term "prophylaxis".

As used herein, an *effective amount* or a *therapeutically effective amount* of an agent defines an amount that can be administered to a subject without excessive toxicity, irritation, allergic response, or other problem or complication, commensurate with a reasonable benefit/risk ratio, but one that is sufficient to provide the desired effect, e.g. the treatment or prophylaxis manifested by a permanent or temporary improvement in the subject's condition. The amount will vary from subject to subject, depending on the age and general condition of the individual, mode of administration and other factors. Thus, while it is not possible to specify an exact effective amount, those skilled in the art will be able to determine an appropriate "effective" amount in any individual case using routine experimentation and background general knowledge. A therapeutic result in this context includes eradication or lessening of symptoms, reduced pain or discomfort, prolonged survival, improved mobility and other markers of clinical improvement. A therapeutic result need not be a complete cure. Improvement may be observed in biological / molecular markers, clinical or observational improvements. In a preferred embodiment, the methods of the invention are applicable to humans, large racing animals (horses, camels, dogs), and domestic companion animals (cats and dogs).

In the context of treatment and effective amounts as defined above, the term *subject* (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, camels, bison, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human. As used herein, the term "equine" refers to mammals of the family *Equidae,* which includes horses, donkeys, asses, kiang and zebra.

"Four branching monomer" refers to a component having a central 4-branching scaffold and four reactive groups capable of reacting acrylate or amine groups. Examples of four-branching monomers include diamine and tetraacrylate components, examples of which are provided above. The scaffold may also be a 4-arm PEG component, a pentaerythritol group, a tetraglycidyl group, or a tetra-substituted silane group. The reactive group may be any acrylamide component (including maleimide), a N-hydroxysuccinimidyl (NHS) component, a thiol component, and an epoxy component. The following are specific examples of four-branching monomers that may be employed in the process and products of the invention:

### 4-arm PEG acrylamide

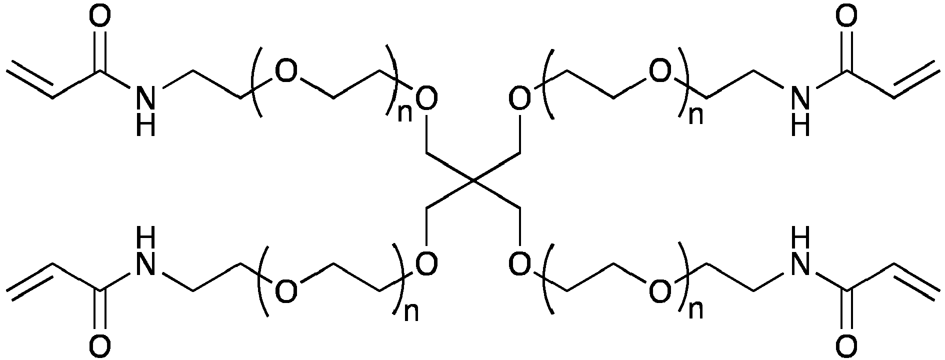

### 4-arm PEG-maleimide

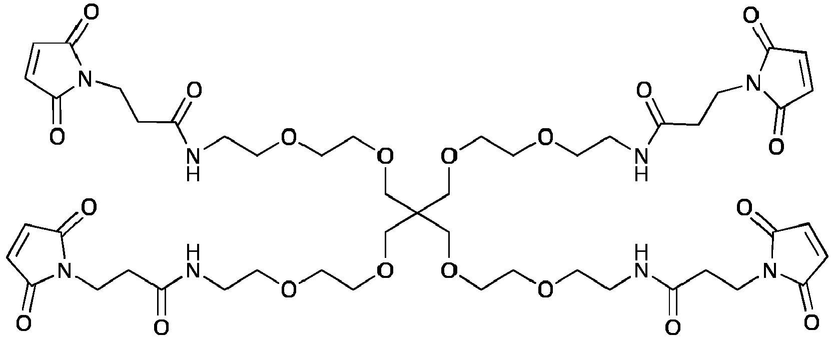

### 4-arm PEG-succinimidyl carbonate NHS

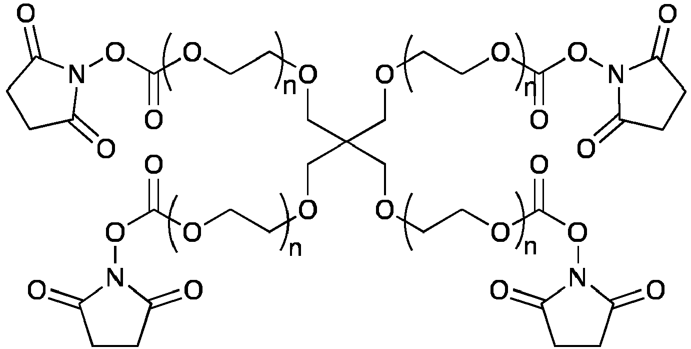

### Tetrathiol components

### Pentaerythritol tetrakis(3-mercaptopropionate):

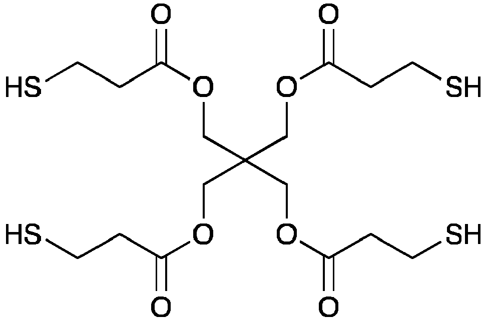

### 4-arm PEG-thiol:

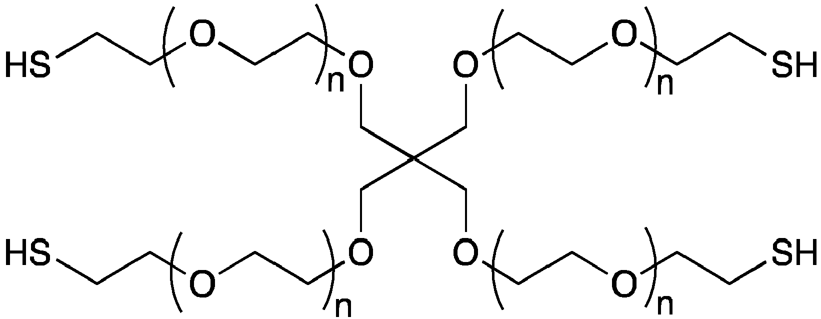

### Tetra(2- mercaptoethyl)silane:

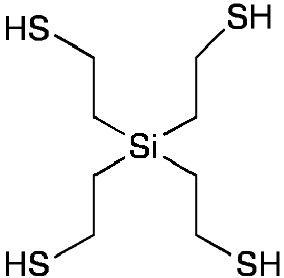

### Tetraepoxy components

### TetraGlycidyl methylenedianiline:

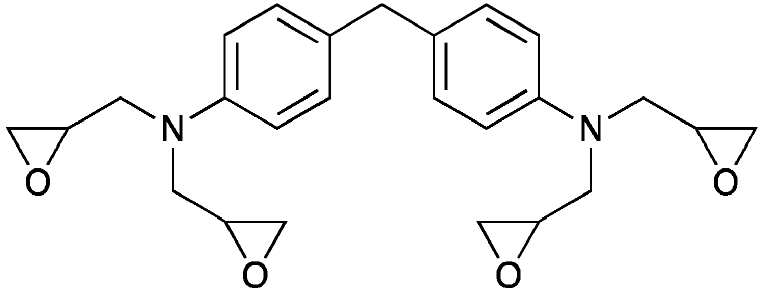

### Tetraglycidyl 1,1'-methylenebis(naphthalene-2,7-diol):

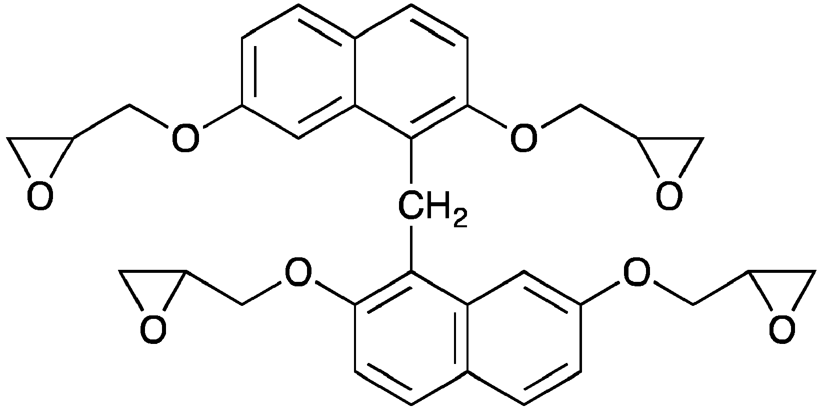

### Pentaerythritol tetraglycidyl ether:

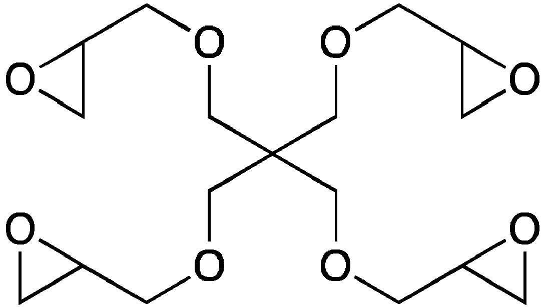

### 4-arm peg epoxide

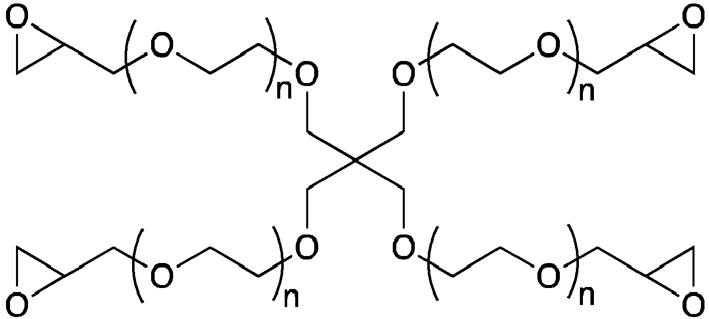

"Linker" means any linker group, including an aryl or alkyl group. Preferred linkers include O, NH, CH₂, alkyl, lower alkyl, alkoxy, lower alkoxy, O-alkyl, CH₂O, CH₂NH, and CH₂NHCOCH₂, CO, COO.

"Diamine component" refers to a moiety having two functional NH2 groups connected by a a linker. "Teraacrylate" refers to a moiety having four functional acrylate groups

"Lower alkyl" means an alkyl group, as defined below, but having from one to ten carbons, more preferable from one to six carbon atoms (eg. "C - C - alkyl") in its backbone structure.

"Alkyl" refers to a group containing from 1 to 8 carbon atoms and may be straight chained or branched. An alkyl group is an optionally substituted straight, branched or cyclic saturated hydrocarbon group. When substituted, alkyl groups may be substituted with up to four substituent groups, at any available point of attachment. When the alkyl group is said to be substituted with an alkyl group, this is used interchangeably with "branched alkyl group". Exemplary unsubstituted such groups include methyl, ethyl, propyl, isopropyl, a-butyl, isobutyl, pentyl, hexyl, isohexyl, 4, 4-dimethylpentyl, octyl, 2,2,4-trimethylpentyl, nonyl, decyl, undecyl, dodecyl, and the like. Exemplary substituents may include but are not limited to one or more of the following groups: halo (such as F, CI, Br, I), Haloalkyl (such as CC13 or CF3), alkoxy, alkylthio, hydroxyl, carboxy (-COOH), alkyloxycarbonyl (-C(O)R), alkylcarbonyloxy (-OCOR), amino (-NH2), carbamoyl (-NHCOOR-or-OCONHR), urea (-NHCONHR-) or thiol (-SH). Alkyl groups as defined may also comprise one or more carbon double bonds or one or more carbon to carbon triple bonds.

"Lower alkoxy" refers to O-alkyl groups, wherein alkyl is as defined hereinabove. The alkoxy group is bonded to the core compound through the oxygen bridge. The alkoxy group may be straight-chained or branched; although the straight-chain is preferred. Examples include methoxy, ethyloxy, propoxy, butyloxy, t-butyloxy, i-propoxy, and the like. Preferred alkoxy groups contain 1-4 carbon atoms, especially preferred alkoxy groups contain 1-3 carbon atoms. The most preferred alkoxy group is methoxy.

"Halogen" means the non-metal elements of Group 17 of the periodic table, namely bromine, chlorine, fluorine, iodine and astatine.

The terms "alkyl", "cycloalkyl", "heterocycloalkyl", "cycloalkylalkyl", "aryl", "acyl", "aromatic polycycle", "heteroaryl", "arylalkyl", "heteroarylalkyl", "amino acyl", "non-aromatic polycycle", "mixed aryl and non-aryl polycycle", "polyheteroaryl", "non-aromatic polyheterocyclic", "mixed aryl and non-aryl polyheterocycles", "amino", and "sulphonyl" are defined in US6,552,065, Column 4, line 52 to Column 7, line 39.

"Halogen" means the non-metal elements of Group 17 of the periodic table, namely bromine, chlorine, fluorine, iodine and astatine.

### Gene therapy/editing

The present invention may be used to edit a portion of the genome of a cell or replace a portion of the genome of a cell with an exogenous DNA insert in an orientation-specific manner.

Thus, the invention may be used to edit or replace a defective portion of a disease-causing gene (i.e. for gene repair), or to insertionally inactivate (i.e. silence) a gene the expression of which is associated with a disease, or to edit or modify a gene for example to delete disease causing mutations or modify or add in residues required for normal functioning of a gene..

Thus, the invention finds application in gene therapy, as herein defined.

Gene therapy according to the invention may target all of the cells in an organism, or may be targeted to a subset of cells (e.g. to selected organs, tissues or cells).

Gene therapy according to the invention may target somatic cells specifically.

Gene therapy according to the invention may exclude the targeting of germ line cells. It may exclude the targeting of totipotent cells. It may exclude the targeting of human embryos.

In cases where gene therapy according to the invention is applied to selected organs, tissues or cells, the method may be applied ex vivo to isolated organs, tissues or cells (e.g. to blood, blood cells, immune cells, bone marrow cells, skin cells, nervous tissue, muscle etc.).

Gene therapy finds application in the treatment of any genetically inherited disorder, particularly those arising from single gene mutations. Thus, gene therapy finds particular application in the treatment of lysosomal storage diseases, muscular dystrophies, cystic fibrosis, Marfan syndrome, sickle cell anaemia, dwarfism, phenylketonuria, neurofibromatosis, Huntington disease, osteogenesis imperfecta, thalassemia and hemochromatosis.

Other diseases which may be suitable for gene therapy according to the invention include diseases and disorders of: blood, coagulation, heterogenous skin disease, cell proliferation and dysregulation, neoplasia (including cancer), inflammatory processes, immune system (including autoimmune diseases), metabolism, liver, kidney, musculoskeletal, neurological, neuronal and ocular tissues.

Exemplary skin diseases include recessive dystrophic epidermolysis bullose (RDEB), a rare heterogenous skin disease caused by biallelic loss-of-function mutations in the COL7A1 gene.

Exemplary blood and coagulation diseases and disorders include: anaemia, bare lymphocyte syndrome, bleeding disorders, deficiencies of factor H, factor H-like 1, factor V, factor VIII, factor VII, factor X, factor XI, factor XII, factor XIIIA, factor XIIIB, Fanconi anaemia, haemophagocytic lymphohistiocytosis, haemophilia A, haemophilia B, haemorrhagic disorder, leukocyte deficiency, sickle cell anaemia and thalassemia.

Examples of immune related diseases and disorders include: AIDS; autoimmune lymphoproliferative syndrome; combined immunodeficiency; HIV -1; HIV susceptibility or infection; immunodeficiency and severe combined immunodeficiency (SCIDs). Autoimmune diseases which can be treated according to the invention include Grave's disease, rheumatoid arthritis, Hashimoto's thyroiditis, vitiligo, type I (early onset) diabetes, pernicious anaemia, multiple sclerosis, glomerulonephritis, systemic lupus E (SLE, lupus) and Sjogren syndrome. Other autoimmune diseases include scleroderma, psoriasis, ankylosing spondilitis, myasthenia gravis, pemphigus, polymyositis, dermomyositis, uveitis, Guillain-Barre syndrome, Crohn's disease and ulcerative colitis (frequently referred to collectively as inflammatory bowel disease (IBD)).

Other exemplary diseases include: amyloid neuropathy; amyloidosis; cystic fibrosis; lysosomal storage diseases; hepatic adenoma; hepatic failure; neurologic disorders; hepatic lipase deficiency; hepatoblastoma, cancer or carcinoma; medullary cystic kidney disease; phenylketonuria; polycystic kidney; or hepatic disease.

Exemplary musculoskeletal diseases and disorders include: muscular dystrophy (e.g. Duchenne and Becker muscular dystrophies), osteoporosis and muscular atrophy.

Exemplary neurological and neuronal diseases and disorders include: ALS, Alzheimer's disease; autism; fragile X syndrome, Huntington's disease, Parkinson's disease, Schizophrenia, secretase related disorders, trinucleotide repeat disorders, Kennedy's disease, Friedrich's ataxia, Machado-Joseph's disease, spinocerebellar ataxia, myotonic dystrophy and dentatorubral pallidoluysian atrophy (DRPLA).

Exemplary ocular diseases include: age related macular degeneration, corneal clouding and dystrophy, cornea plana congenital, glaucoma, leber congenital amaurosis and macular dystrophy.

Gene therapy according to the invention finds particular application in the treatment of lysosomal storage disorders. Listed below are exemplary lysosomal storage disorders and the corresponding defective enzymes:

| | |
|---|---|
| Pompe disease: | Acid alpha-glucosidase |
| Gaucher disease: | Acid beta-glucosidase or glucocerebrosidase |
| Fabry disease: | alpha-Galactosidase A |
| GMI-gangliosidosis: | Acid beta-galactosidase |
| Tay-Sachs disease: | beta-Hexosaminidase A |
| Sandhoff disease: | beta-Hexosaminidase B |
| Niemann-Pick disease: | Acid sphingomyelinase |
| Krabbe disease: | Galactocerebrosidase |
| Farber disease: | Acid ceramidase |
| Metachromatic leukodystrophy: | Arylsulfatase A |
| Hurler-Scheie disease: | alpha-L-Iduronidase |
| Hunter disease: | Iduronate-2-sulfatase |
| Sanfilippo disease A: | Heparan N-sulfatase |
| Sanfilippo disease B: | alpha-N-Acetylglucosaminidase |
| Sanfilippo disease C: | Acetyl-CoA: alpha-glucosaminide N-acetyltransferase |
| Sanfilippo disease D: | N-Acetylglucosamine-6-sulfate sulfatase |
| Morquio disease A: | N-Acetylgalactosamine-6-sulfate sulfatase |
| Morquio disease B: | Acid beta-galactosidase |
| Maroteaux-Lamy disease: | Arylsulfatase B |
| Sly disease: | beta-Glucuronidase |
| alpha-Mannosidosis: | Acid alpha-mannosidase |
| beta-Mannosidosis: | Acid beta-mannosidase |
| Fucosidosis: | Acid alpha-L-fucosidase |
| Sialidosis: | Sialidase |
| Schindler-Kanzaki disease: | alpha-N-acetylgalactosaminidase |

Gene therapy according to the invention also finds particular application in the treatment of proteostatic diseases including both aggregative and misfolding proteostatic diseases, for example prion diseases, various amyloidoses and neurodegenerative disorders (e.g. Parkinson's disease, Alzheimer's disease and Huntington's disease), certain forms of diabetes, emphysema, cancer and cystic fibrosis.

Gene therapy according to the invention finds particular application in the treatment of cystic fibrosis. Cystic fibrosis occurs when there is a mutation in the CFTR gene leading to reduced ion channel activity (via increased clearance of the misfolded CFTR proteins).

Gene therapy according to the invention finds particular application in the treatment of expanded CAG repeat diseases. These diseases stem from the expansion of CAG repeats in particular genes with the encoded proteins having corresponding polyglutamine tracts which lead to aggregation and accumulation in the nuclei and cytoplasm of neurons. Aggregated amino-terminal fragments of mutant huntingtin are toxic to neuronal cells and are thought to mediate neurodegeneration. Examples include Huntington's disease (HD), which is characterized by selective neuronal cell death primarily in the cortex and striatum. CAG expansions have also been found in at least seven other inherited neurodegenerative disorders, including for example spinal and bulbar muscular atrophy (SBMA), Kennedy's disease, some forms of amyotrophic lateral sclerosis (ALS), dentatorubral pallidoluysian atrophy (DRPLA) and spinocerebellar ataxia (SCA) types 1, 2, 3, 6 and 7.

Gene therapy according to the invention finds particular application in the treatment of any neoplasia, including proliferative disorders, benign, pre-cancerous and malignant neoplasia, hyperlasia, metaplasia and dysplasia. The invention therefore finds application in the treatment of proliferative disorders which include, but are not limited to cancer, cancer metastasis, smooth muscle cell proliferation, systemic sclerosis, cirrhosis of the liver, adult respiratory distress syndrome, idiopathic cardiomyopathy, lupus erythematosus, retinopathy (e.g. diabetic retinopathy), cardiac hyperplasia, benign prostatic hyperplasia, ovarian cysts, pulmonary fibrosis, endometriosis, fibromatosis, harmatomas, lymphangiomatosis, sarcoidosis and desmoid tumours. Neoplasia involving smooth muscle cell proliferation include hyperproliferation of cells in the vasculature (e.g. intimal smooth muscle cell hyperplasia, restenosis and vascular occlusion, including in particular stenosis following biologically- or mechanically-mediated vascular injury, such as angioplasty). Moreover, intimal smooth muscle cell hyperplasia can include hyperplasia in smooth muscle other than the vasculature (e.g. blockage of the bile duct, bronchial airways and in the kidneys of patients with renal interstitial fibrosis). Non-cancerous proliferative disorders also include hyperproliferation of cells in the skin such as psoriasis and its varied clinical forms, Reiter's syndrome, pityriasis rubra pilaris and hyperproliferative variants of disorders of keratinization (including actinic keratosis, senile keratosis and scleroderma). Particularly preferred is the treatment of malignant neoplasia (cancer).

The term "pharmaceutically acceptable excipient" refers to a diluent, adjuvant, excipient, or vehicle with which the polyplex is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

### EXAMPLE 1 - Y Polymer (4-branching diamine)

An embodiment of a 4-branching diamine hyperbranched cationic polymer was prepared according to Scheme 1 below, using BDA, EDA (or HMDA), S5 and DA (or DATA) monomers.

To synthesize the cationic Y polymer the monomers: BDA, EDA (or HMDA), S5, and DA (or DATA) were used. The Y polymer was formed through an "A2+B4+C2" Michael addition strategy using the copolymerization of commercially available monomers. Each selected monomer within the reaction system plays a pivotal role in the final Y4 polymer. Diamine monomer (B4) was employed as the branching unit to generate the highly branched polymer through combination with linear diacrylate monomer (A2). Further post synthesis modification involved end capping polymer terminal groups with additional (Amine monomer, C2) to remove any unreacted vinyl groups. Monomers were added into a round bottomed flask with a magnetic stirring bar. The flask was placed partially submerged in an oil bath and polymerization reactions were carried out at 90°C. Gel permeation chromatography (GPC) was used to track the progression of the polymer synthesis reaction by measuring molecular weight, conversion and PDI. Upon polymer molecular weight (Mw) approaching 10-20 kDa the reaction was stopped by removing from heat and diluted with DMSO. Y polymers chain termination was achieved by reacting the polymer solution with the amine end capping agent for 48 hrs at room temperature. Post endcapping, Y polymers were purified by precipitating in excess diethyl ether twice so as to remove any remaining residual monomers, unreacted end capping agents and small oligomers. To achieve the final product, Y polymers were dried in a vacuum oven for 48 hrs to remove remaining solvents.

### EXAMPLE 2 - P Polymer (4-branching tetraacrylate)

An embodiment of a 4-branching tetraacrylate hyperbranched cationic polymer was prepared according to Scheme 2 below, using BDA, PTTA (or DTTA), S5 and DA (or DATA) monomers. P1 polymer (7kDa) and P2 polymer (10 kDa).

BDA, PTTA (or DTTA), and S5 were mixed into a flask with DMSO as the solvent. The reaction was performed at 90 °C until the target Mw was achieved. The reaction was stopped by removing the reaction flask from heat and cooled with ice. Endcapping monomer DA (or DATA) was added into the flask with DMSO to react with the acrylate residual for 48 hrs at room temperature. Afterwards, the reaction mixture was precipitated into excess amount of diethyl ether twice to remove the monomers and oligomers. The P1 and P2 polymers were achieved by drying in a vacuum oven with 7 kDa and 10 kDa Mw, respectively.

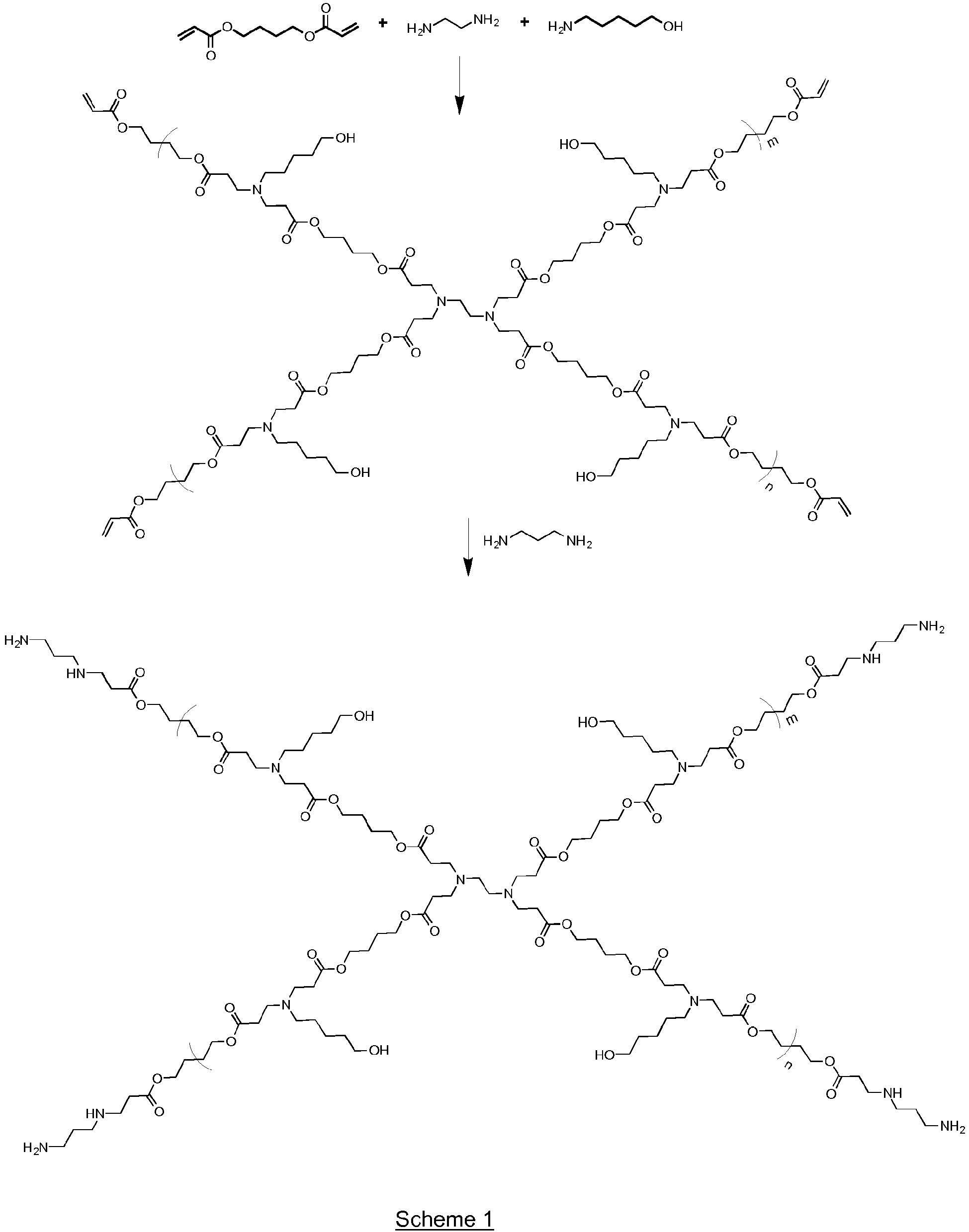

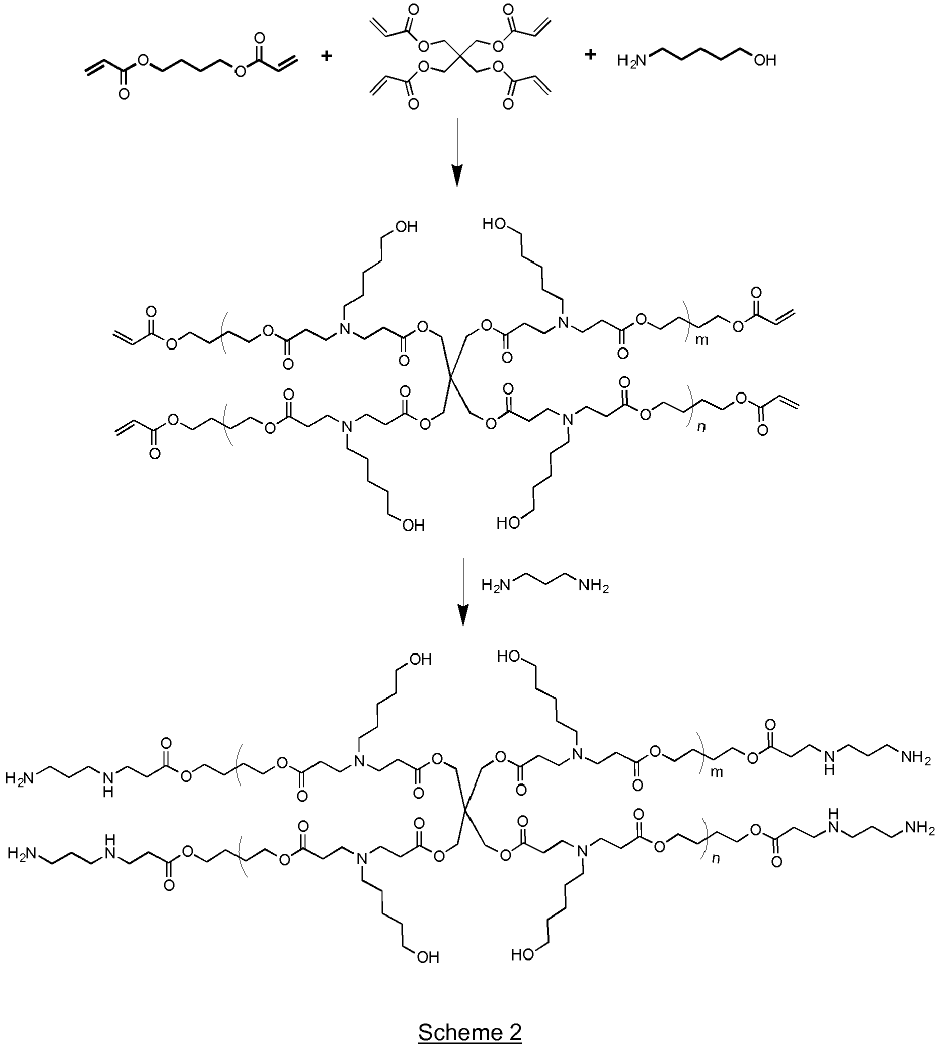

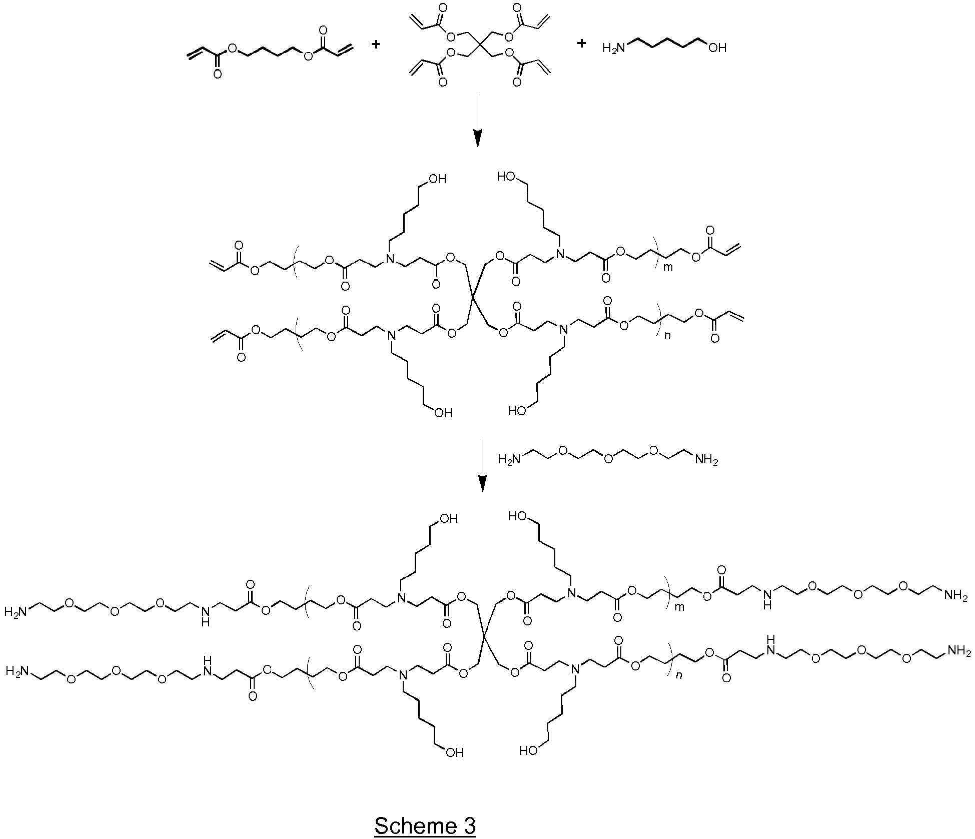

### Proton Nuclear Magnetic Resonance (¹H-NMR) Measurement:

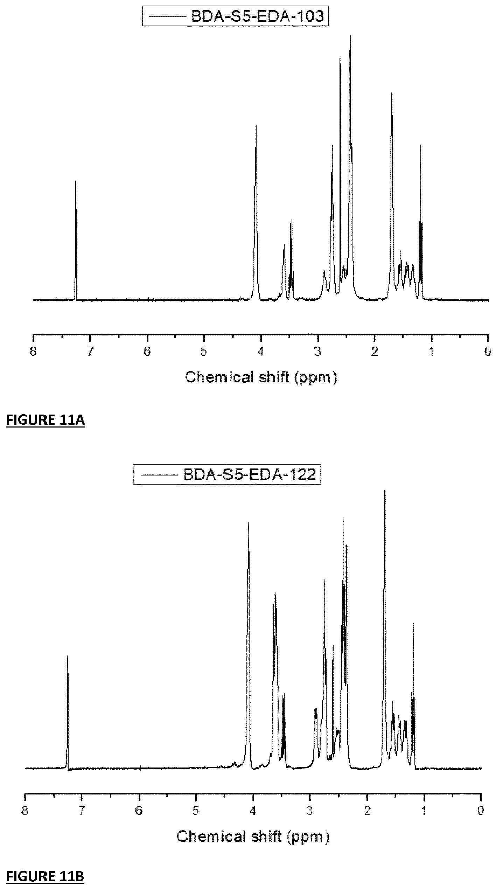

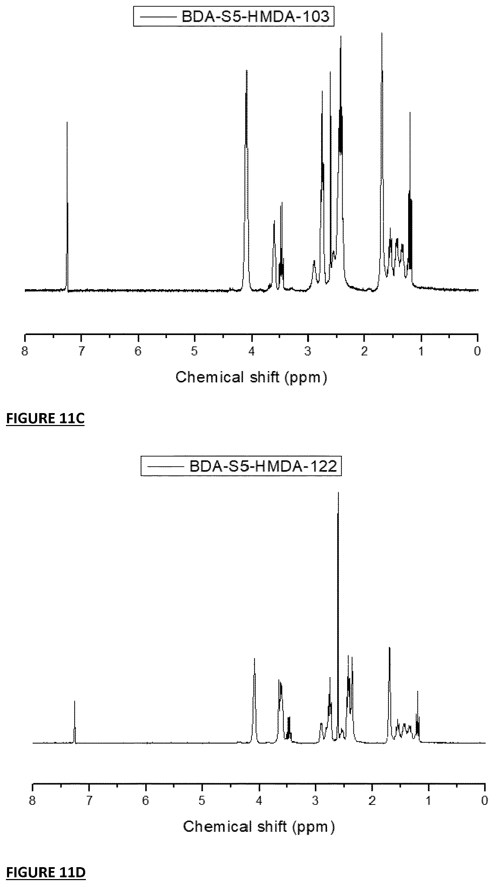

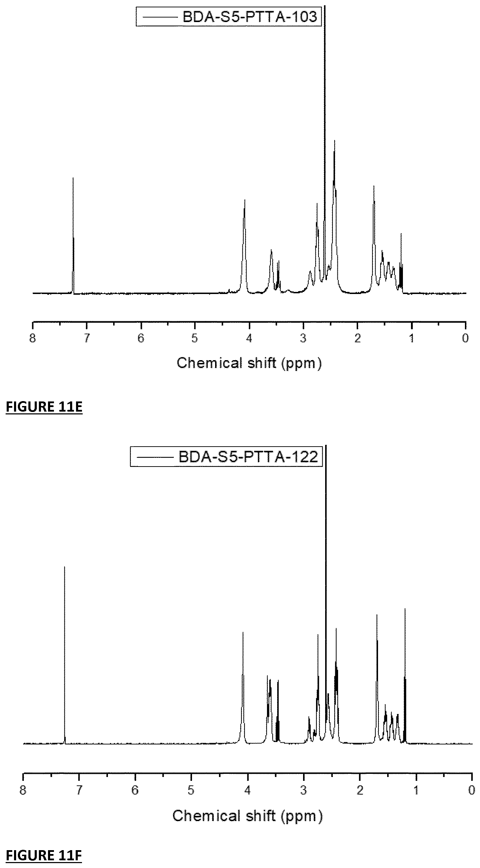

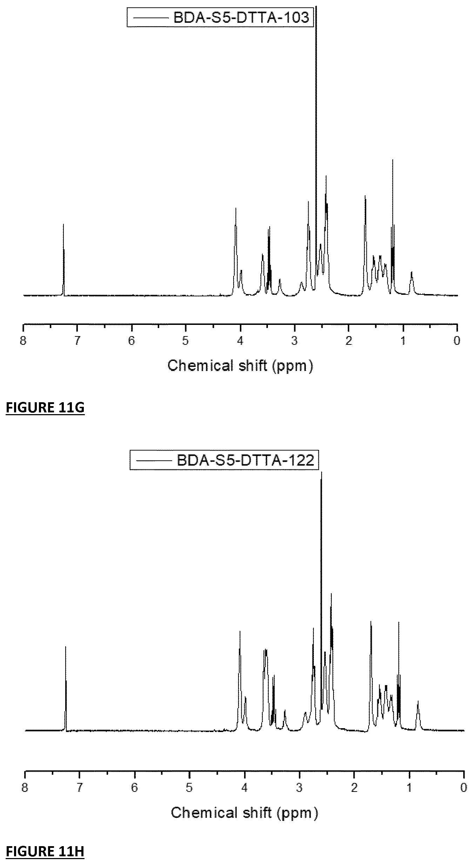

The chemical structure of Y4 and P polymers was confirmed by ¹H-NMR. The polymer was dissolved in deuterated chloroform (CDCl₃) and measurements were carried out on a 300 MHz Bruker NMR equipment. All chemical shifts were reported in ppm relative to tetramethylsilane (TMS). Figure 11.

### Molecular Weight Determination by Gel Permeation Chromatography (GPC):

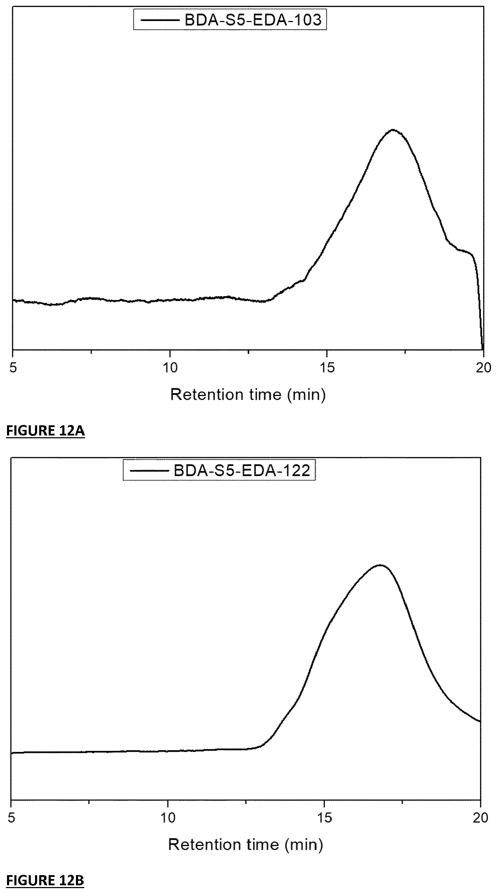

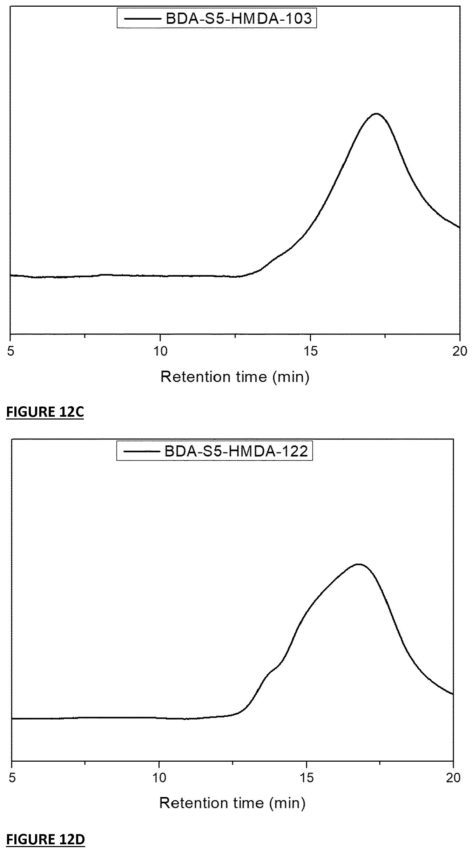

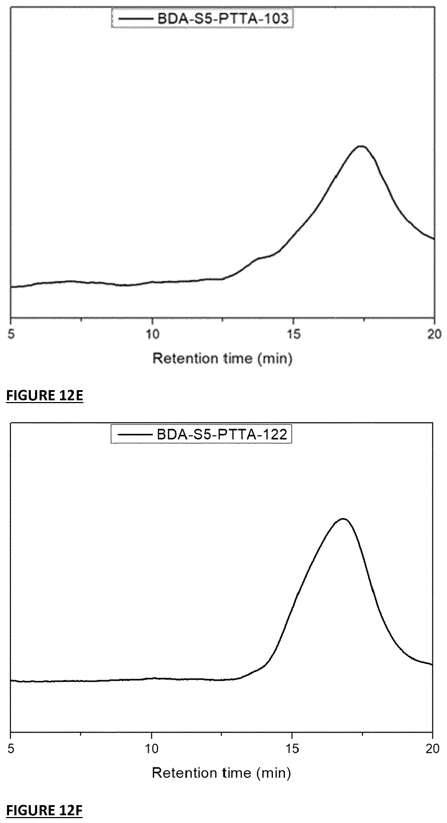

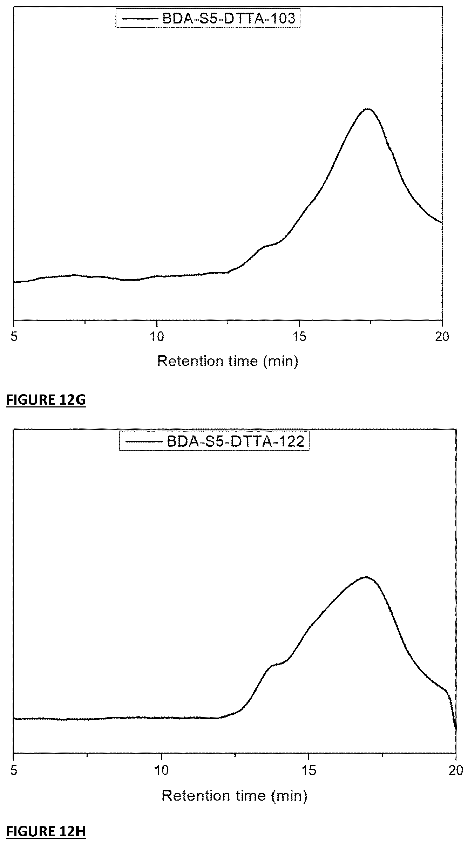

Molecular weight and polydispersity of the Y4 and P polymers were measured by GPC. A 50 µL of the reaction mixture was collected and diluted into 1 mL DMF, filtered through a 0.22 µm filter and then analyzed using an Agilent 1260 GPC/SEC instrument equipped with a triple detector at 60 °C. DMF was used as the mobile phase and the flow rate was 1 mL per min. Poly(methyl methacrylate) was used as the standard sample for GPC calibration. Figure 12.

### Synthesis of Polyplexes involving Polymers and Plasmid DNA

Polyplexes are formed by first preparing aqueous polymer and DNA solutions in a suitable solvent, for example 25mM sodium acetate, 1:1 v/v ratio of dissolved DNA and dissolved polymer are mixed together, polymer-DNA solution is incubated at room temperature for 10 min to allow polyplex formation prior to use. The aqueous polyplex solution should be mixed vigorously to ensure solution homogeneity.

### Cell Viability Studies

Evaluation of cell cytotoxicity induced by different polyplex conditions has been assessed using the alamarBlue™ assay, which provided a quantitative measurement of cell proliferation and metabolic health. Cell viability has been assessed 48-72 hrs post transfection experiments in cells. Culture media is removed from cells in a well plate and cells are washed with (hanks balanced salt solution) HBSS per well. Following this, 100 µl of alamarBlue™ working solution (10% alamarBlue™ in HBSS) is added to each well and allowed to incubate under normal cell culture conditions for 2 hrs protected from light. After incubation, the alamarBlue™ solution is transferred to a fresh flat bottomed 96 well plate and absorbance at 570 nm and 600 nm is recorded on a SpectraMax M3 multi-plate reader. Wells containing alamarBlue™ reagent only are subtracted from each sample as a background reading. Untreated cells are used to normalize fluorescence values and plotted as 100% viable.

### Cell transfection Studies for polyplexes of polymer and DNA

Cells are seeded 24hr-48hrs prior to transfections to allow attachment to well plates and flasks. Cells are seeded at optimized cell densities. On the day of transfection, polymer-DNA complexes are prepared and after complexation, are mixed with the appropriate cell media such that the final polyplex solution is no more than 20% of the overall media volume. Cell media containing polymer-DNA complexes are added to cells and after 4hrs is removed and replaced with fresh media to remove complexes.

### RNP System

Both crRNAs and tracrRNA are diluted to 100µM with nuclease free duplex buffer with HiFi Cas9 nuclease used at stock concentration of 62µM as per manufacturers guidelines. RNP complexes are prepared such that sgRNA(crRNA+tracrRNA): Cas9 molar ratio is 1-10: 1. Master mixes are heated for 5min at 95°C in a thermocycler to anneal crRNA and tracrRNA. Following this, they are removed from heat and allowed to cool to room temperature on bench top. To each master mix HiFi Cas9 nuclease is added and allowed to complex for 15min at room temperature, protected from light. Polymer:RNP polyplexes (ribopolyplexes) are prepared for transfection in a similar manner to that of plasmid DNA based transfections. Polymer is diluted in 25mM sodium acetate buffer to desired concentrations as previously described. Equal amounts of each RNP master mix 1 and 2 are used for every transfection. To calculate the w/w ratio for the polymer, the entire weight of the RNP complex is used. To form complexes, polymer solutions are mixed with RNP solutions at a 1:1 v/v ratio. After mixing together by pipetting up and down, complexes are incubated at room temperature for 15 min to allow polymer-RNP interactions. Once incubation is completed, ribopolyplex solutions are ready to be diluted in appropriate cell culture media and added to cells. 4 hrs after transfection, media is changed and replaced with fresh culture media. An ATTO 550 nm fluorophore on the tracrRNA is used an indicator of transfection efficiency.

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. A hyperbranched polymer made by reacting together:
(i) a four-branching monomer;
(ii) a diacrylate component of formula (I) 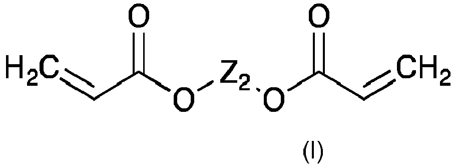 wherein Z2 is a linear or branched carbon chain of 1 to 30 carbon atoms, a linear or branched heteroatom-containing carbon chains of 1 to 30 atoms, a carbocycle containing 3 to 30 carbon atoms, or a heterocycle containing 3 to 30 atoms; wherein Z2 is unsubstituted or substituted with at least one of a halogen, a hydroxyl, an amino group, a suifonyl group, a sulphonamide group, a thiol, a C1-C6 alkyl, a C1-C0 alkoxy, a C1-C6 ether, a C1-C6 thioether, a C1-C6 sulfone, a C1-C6 sulfoxide, a C1-C6 primary amide, a C1-C6 secondary amide, a halo C1-C5 alkyl, a carboxyl group, a cyano group, a nitro group, a nitroso group, -OC(0)NR'R', - N(R')C(0)NR'R, -N(R')C(0)0-C1-C6 alkyl, C3-C6 cycloalkyl, C3-C6 heterocyclyl, C2-C5 heteroaryl and C6-C10 aryl; wherein each R' is independently selected, from the group consisting of hydrogen and Ci-C6 alkyl;
(iii) a first amine component comprising 3 to 20 atoms,
wherein said amine component is unsubstituted or substituted with at least one of a halogen, a hydroxyl, an amino group, a sulfonyl group, a sulphonamide group, a thiol, a C1-C6 alkyl, a C1-C6 alkoxy, a C1-C6 ether, a C1-C6 thioether, a C1-C6 sulfone, a C1-C6 sulfoxide, a C1-C6 primary amide, a C1-C6 secondary amide, a halo C1-C6 alkyl, a carboxyl group, a cyano group, a nitro group, a nitroso group, - OC(0)NR'R', -N(R')C(0)NR'R, -N(R')C(0)0-C1-C6 alkyl, C3-C6 cycloalkyl, C3-C6 heterocyclyl, C2-C5 heteroaryl and C6-C10 aryl; wherein each R' is independently selected, from the group consisting of hydrogen and C1-C6 alkyl; and
(iv) a second amine component comprising 3 to 20 atoms,
wherein said amine component is unsubstituted or substituted with at least one of a halogen, a hydroxyl, an amino group, a sulfonyl group, a sulphonamide group, a thiol, a C1-C6 alkyl, a C1-C6 alkoxy, a C1-C6 ether, a C1-C6 thioether, a C1-C6 sulfone, a C1-C6 sulfoxide, a C1-C6 primary amide, a C1-C6 secondary amide, a halo C1-C6 alkyl, a carboxyl group, a cyano group, a nitro group, a nitroso group, - OC(0)NR'R', -N(R')C(0)NR'R, -N(R')C(0)0-C1-C6 alkyl, C3-C6 cycloalkyl, C3-C6 heterocyclyl, C2-C5 heteroaryl and C6-C10 aryl; wherein each R' is independently selected, from the group consisting of hydrogen and C1-C6 alkyl.

2. A hyperbranched polymer according to Claim 1, in which the diacrylate and first amine components are reacted together to form a first oligomer, the first oligomer and second amine component are reacted together to form a second oligomer, and the second oligomer and four branching monomer are reacted together to form the hyperbranched polymer.

3. A hyperbranched polymer according to Claim 1, in which the four-branching monomer, diacrylate component, and first amine are reacted together in a Michael addition reaction to form a first polymer, and the first polymer and second amine component are reacted together in a Michael addition reaction to form the hyperbranched polymer.

4. A hyperbranched polymer according to any preceding Claim, in which the four-branching monomer is selected from a diamine component, a tetraacrylate component, a tetra acrylamide component, a tetrathiol component, a tetraepoxy component, and a tetra-substituted silane.

5. A hyperbranched polymer according to Claim 4, in which the four-branching monomer is selected from the group consisting of:

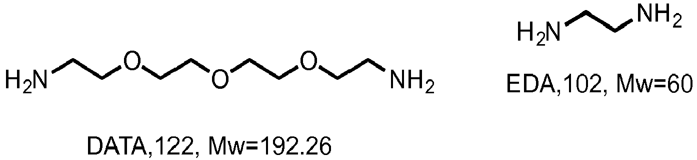

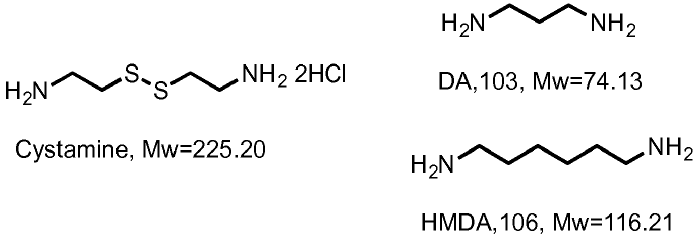

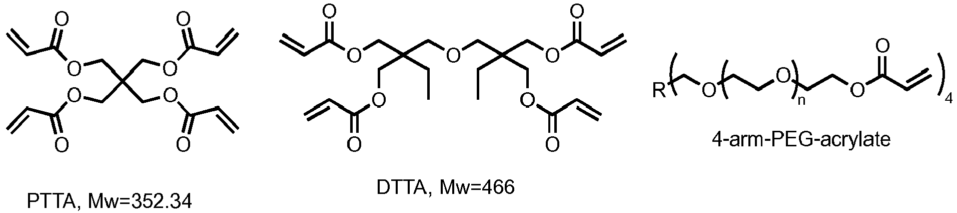

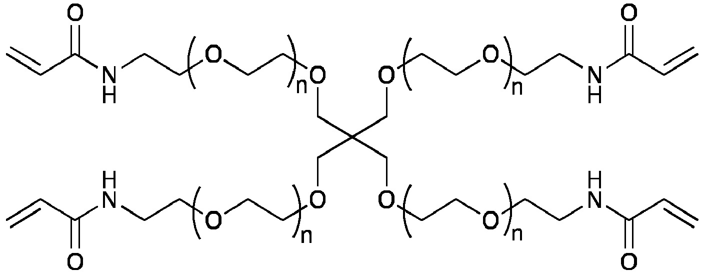

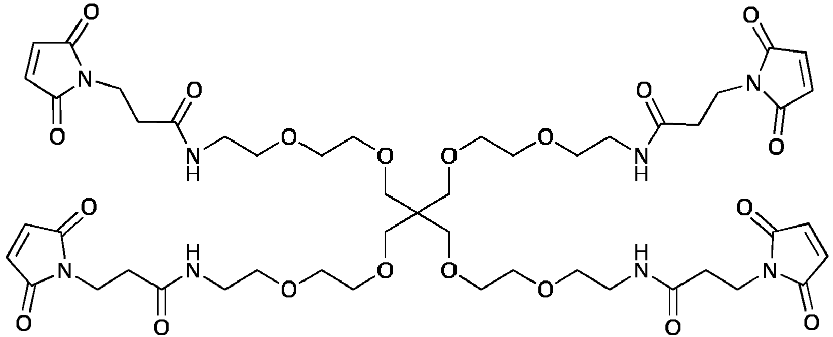

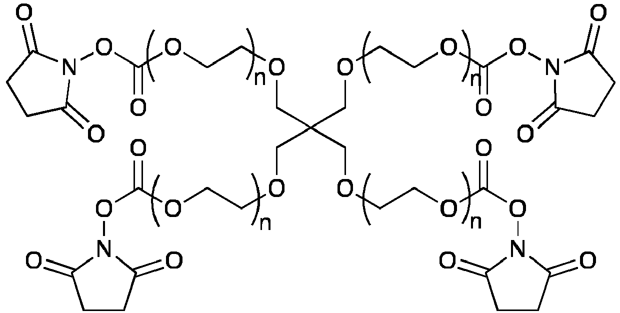

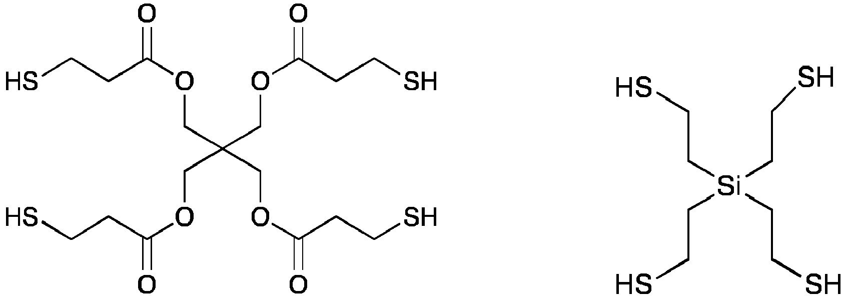

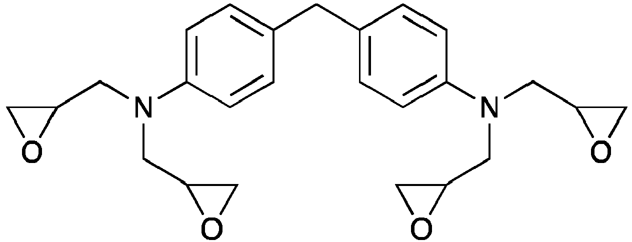

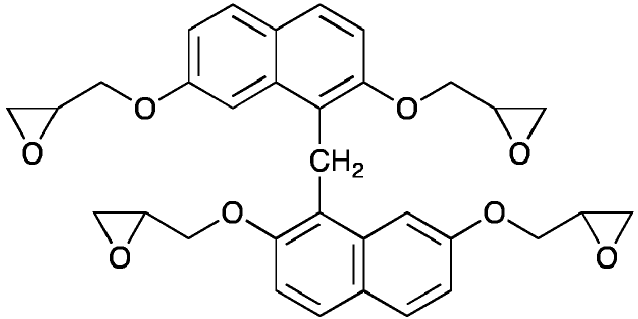

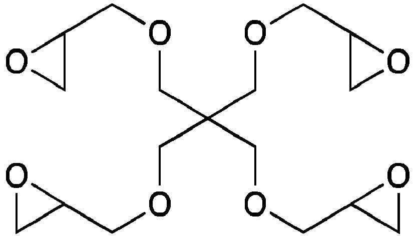

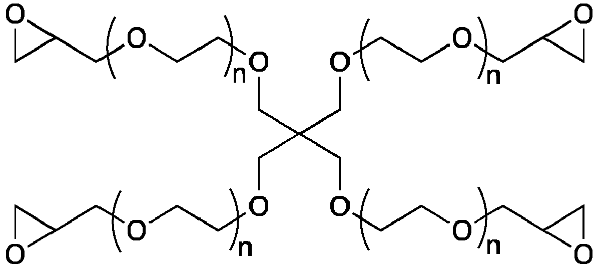

6. A hyperbranched polymer according to any preceding Claim, in which the diacrylate component of formula (I) is selected from

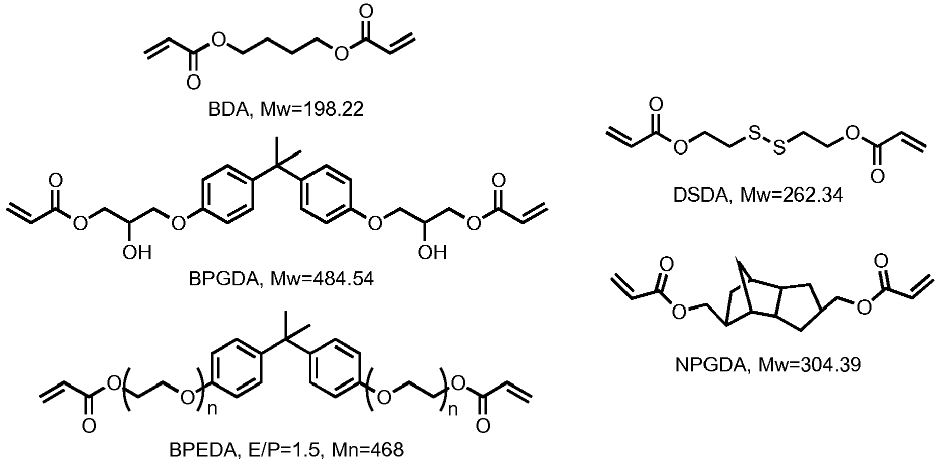

7. A hyperbranched polymer according to any preceding Claim, in which the first amine component is selected from:

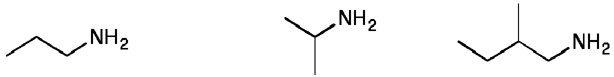

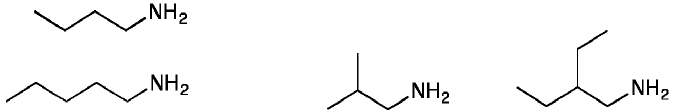

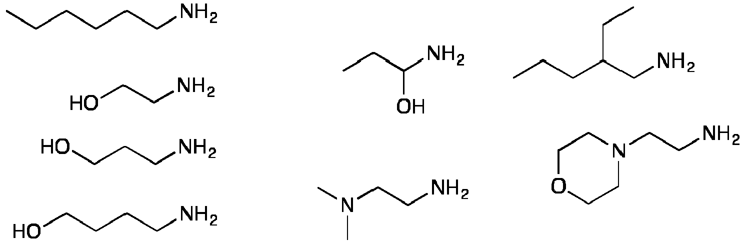

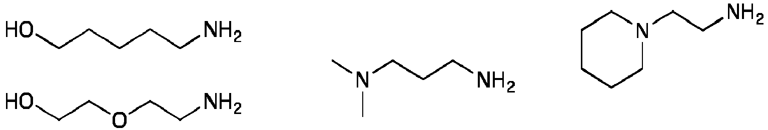

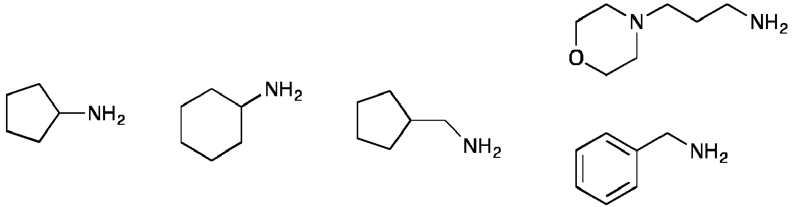

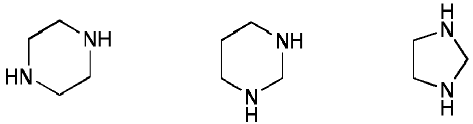

8. A hyperbranched polymer according to any preceding Claim, in which the second amine component is a diamine component having a structure NH₂-L₄-NH₂ or is selected from:

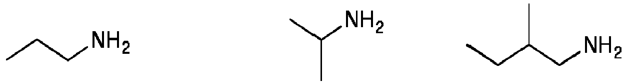

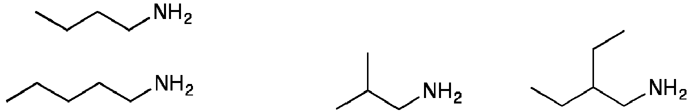

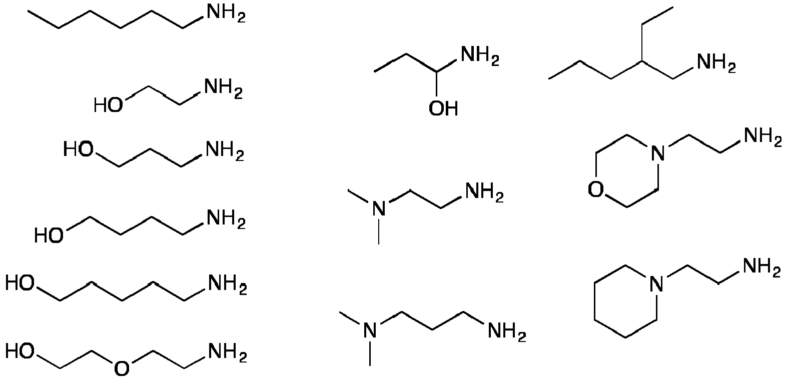

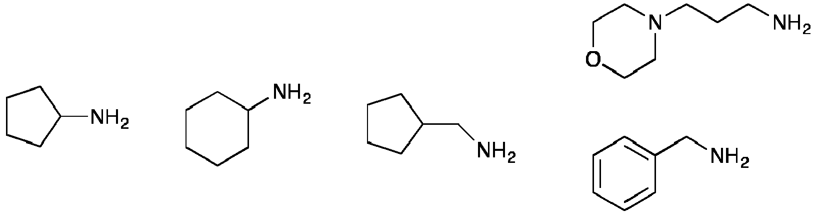

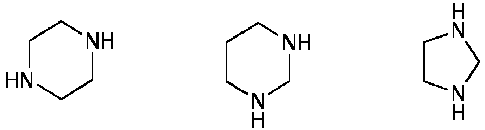

9. A hyperbranched polymer according to any preceding Claim, in which the second amine component is selected from:

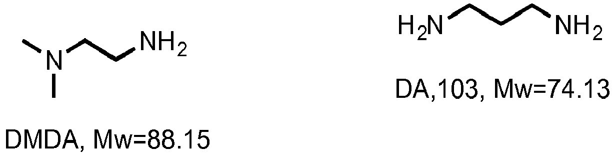

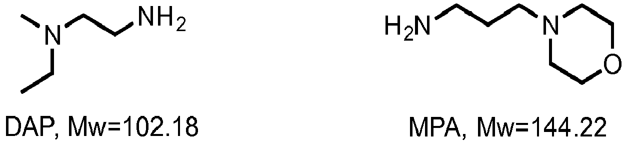

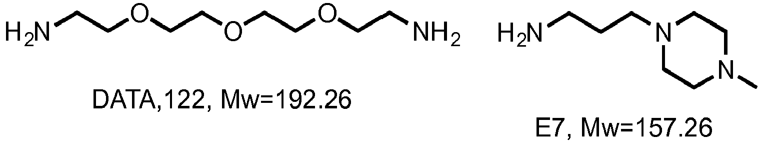

10. A polyplex composition comprising a hyperbranched polymer of any preceding Claim and a nucleic acid component complexed together.

11. A polyplex composition according to Claim 10, in which the nucleic acid component is selected from a nucleic acid, a nucleic acid expression system, a plasmid, a nanoplasmid, a minicircle, and a ribonucleoprotein gene editing system.

12. A polyplex composition according to Claim 10 or 11, in which the nucleic acid component is a ribonucleoprotein gene editing system.

13. A polyplex composition according to any of Claims 10 to 12, in which the composition has a particle size of 100-500 nm.

14. A polyplex composition of any of Claims 10 to 13, for use as a medicament.

15. A method of forming a polymer comprising a step of reacting together:
(i) a four-branching monomer;
(ii) a diacrylate component of formula (I) 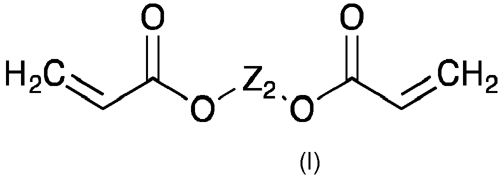 wherein Z2 is a linear or branched carbon chain of 1 to 30 carbon atoms, a linear or branched heteroatom-containing carbon chains of 1 to 30 atoms, a carbocycle containing 3 to 30 carbon atoms, or a heterocycle containing 3 to 30 atoms; wherein Z2 is unsubstituted or substituted with at least one of a halogen, a hydroxyl, an amino group, a suifonyl group, a sulphonamide group, a thiol, a C1-C6 alkyl, a C1-C0 alkoxy, a C1-C6 ether, a C1-C6 thioether, a C1-C6 sulfone, a C1-C6 sulfoxide, a C1-C6 primary amide, a C1-C6 secondary amide, a halo C1-C5 alkyl, a carboxyl group, a cyano group, a nitro group, a nitroso group, -OC(0)NR'R', - N(R')C(0)NR'R, -N(R')C(0)0-C1-C6 alkyl, C3-C6 cycloalkyl, C3-C6 heterocyclyl, C2-C5 heteroaryl and C6-C10 aryl; wherein each R' is independently selected, from the group consisting of hydrogen and Ci-C6 alkyl
(iii) a first amine component comprising 3 to 20 atoms,
wherein said amine component is unsubstituted or substituted with at least one of a halogen, a hydroxyl, an amino group, a sulfonyl group, a sulphonamide group, a thiol, a C1-C6 alkyl, a C1-C6 alkoxy, a C1-C6 ether, a C1-C6 thioether, a C1-C6 sulfone, a C1-C6 sulfoxide, a C1-C6 primary amide, a C1-C6 secondary amide, a halo C1-C6 alkyl, a carboxyl group, a cyano group, a nitro group, a nitroso group, - OC(0)NR'R', -N(R')C(0)NR'R, -N(R')C(0)0-C1-C6 alkyl, C3-C6 cycloalkyl, C3-C6 heterocyclyl, C2-C5 heteroaryl and C6-C10 aryl; wherein each R' is independently selected, from the group consisting of hydrogen and C1-C6 alkyl; and
(iv) a second amine component comprising 3 to 20 atoms,
wherein said amine component is unsubstituted or substituted with at least one of a halogen, a hydroxyl, an amino group, a sulfonyl group, a sulphonamide group, a thiol, a C1-C6 alkyl, a C1-C6 alkoxy, a C1-C6 ether, a C1-C6 thioether, a C1-C6 sulfone, a C1-C6 sulfoxide, a C1-C6 primary amide, a C1-C6 secondary amide, a halo C1-C6 alkyl, a carboxyl group, a cyano group, a nitro group, a nitroso group, - OC(0)NR'R', -N(R')C(0)NR'R, -N(R')C(0)0-C1-C6 alkyl, C3-C6 cycloalkyl, C3-C6 heterocyclyl, C2-C5 heteroaryl and C6-C10 aryl; wherein each R' is independently selected, from the group consisting of hydrogen and C1-C6 alkyl.
